(19) Europäisches Patentamt
European Patent Office
Office européen des brevets

(11) EP 1 445 303 A2

(12) EUROPEAN PATENT APPLICATION

(43) Date of publication:
**11.08.2004 Bulletin 2004/33**

(51) Int Cl.[7]: **C11D 3/00**

(21) Application number: **04009337.9**

(22) Date of filing: **13.10.1995**

(84) Designated Contracting States:
**DE ES FR GB IT**

(30) Priority: **14.10.1994 JP 24911594**
**25.10.1994 JP 26008094**

(62) Document number(s) of the earlier application(s) in accordance with Art. 76 EPC:
**95116186.8 / 0 707 059**

(71) Applicant: **KAO CORPORATION**
**Chuo-ku, Tokyo (JP)**

(72) Inventors:
- **Sakata, Yushi**
  **Wakayama-shi Wakayama (JP)**
- **Inokoshi, Junichi**
  **Wakayama-shi Wakayama (JP)**
- **Nishimoto, Uichiro**
  **Wakayama-shi Wakayama (JP)**
- **Kato, Tohru**
  **Wakayama-shi Wakayama (JP)**
- **Osés, Maria Jose Bermejo**
  **08210 Barberà del Vallès Barcelona (ES)**
- **Okabe, Kazuhiko**
  **08210 Barberà del Vallès Barcelona (ES)**
- **Sotoya, Kohshiro**
  **Naga-gun Wakayama (JP)**

(74) Representative: **HOFFMANN - EITLE**
**Patent- und Rechtsanwälte**
**Arabellastrasse 4**
**81925 München (DE)**

Remarks:
This application was filed on 20 - 04 - 2004 as a divisional application to the application mentioned under INID code 62.

# (54) Liquid softener composition

(57) The invention provides a liquid softener composition comprising the following component (2-A) and the following component (2-B) wherein the weight ratio of the component (2-A) to the component (2-B) ranges from 2/1 to 1/9:

[component (2-A)]

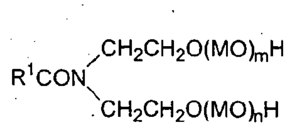

[component (2-B)]

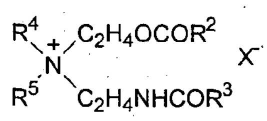

[wherein

| | |
|---|---|
| $R^1$, $R^2$ and $R^3$: | each alkyl or alkenyl group having 7 to 23 carbon atoms, |
| M: | an alkylene group having 2 or 3 carbon atoms, and m and |

EP 1 445 303 A2

n: each a number of 0 or above, provided that the sum of m and n is 0 to 4 on the average.
$R^4$ and $R^5$: each an alkyl group having 1 to 4 carbon atoms, and
$X^-$ a halogen ion or $R^4SO_4$ wherein $R^4$ is as defined above].

## Description

### Background of the Invention

### Field of the Invention

The present invention relates to a liquid softener composition for clothes which is applicable to a rinsing bath for the washing and can impart excellent softness, antistatic properties and water absorption properties to various textiles.

### Prior Art

Clothes have a tendency to be stiffened owing to the washing-away of textile auxiliaries or the degradation of fiber itself during repeated wear and washing to result in uncomfortable hand. Therefore, softeners which can impart softness and antistatic properties to textile have recently been used in many households.
Such a softener lowers the frictional coefficient of fiber surface by the lubricating effect due to the lipophilic moiety of the base material molecule of the softener adsorbed on the fiber surface to thereby exhibit a softening effect. When a textile is treated with a softener containing a base material having an excellent softening effect, however, the resulting textile cannot avoid becoming greasy to the touch. In particular, when underwear which comes into direct contact with the skin (such as cotton knit underwear or nylon tricot slip) or a towel which comes into direct contact with the skin and the hand of which the user is sensitive to was treated with such a softener, the resulting underwear or towel had a problem of being greasy to the touch, though the greasiness of the textile treated with the softener varies depending upon the kind of fiber, the method of knitting and so forth. Further, when the softener of the prior art was used in the treatment of clothes in a higher concentration, a higher softening effect was attained but the greasiness of the resulting clothes was more significant. Accordingly, it was believed with respect to the softener composition of the prior art that there was a correlation between softening effect and greasiness.
Under these circumstances, there have been proposed a softener for clothes comprising a neutralization or quaternization product of an amine compound having ester and amide groups in its molecule and an adduct of fatty acid derivative with alkylene oxide (see Japanese Patent Laid-Open No. 57632/1994), a softener usable in rising after washing which comprises an alkoxylated natural fat or oil (see Japanese Patent Laid-Open No. 10263/1994) and so forth. When cloth is treated with these softener compositions, no excellent softening effect is found in some cases, while excellent softening effect is found with undesirable greasy touch in the other cases. No satisfactory softener has been developed as yet.

### Disclosure of the Invention

The present invention aims at providing a liquid softener composition which is applicable to a rinsing bath for the washing and can impart excellent softness to cloth without making the cloth greasy to the touch.
The inventors of the present invention have intensively studied to attain the above aim, and have found as a result of the studies that the above aim can be attained by combining severely selected components. The present invention has been accomplished on the basis of this finding.
The present invention discloses (1) a composition comprising either a mixture of (A) an adduct of glycerol with (B) a quaternary ammonium salt or (A) an adduct of glycerol, and water (2) a composition comprising (2-A) an adduct of amide and (B) a quaternary salt, (3) a process for the preparation of a quaternary salt and (4) an improvement in the preparation of a quaternary salt.
Namely, the invention (1) provides a liquid softener composition comprising the following softener base material (1) or (2) and water:

[softener base material (1)]

a mixture comprising a compound represented by the following general formula (I) (hereinafter referred to as "compound (I)"), a compound represented by the following general formula (II) (hereinafter referred to as "compound (II)"), a compound represented by the following general formula (III) (hereinafter referred to as "compound (III)") and a compound represented by the following general formula (IV) (hereinafter referred to as "compound (IV)"), provided that

the weight ratio of the compound (I) to the sum total of the compounds (I), (II), (III) and (IV) ranges from 0.040 to 0.327,

the weight ratio of the compound (II) to the sum total of the compounds (I), (II), (III) and (IV) ranges from 0.298

to 0.469,

the weight ratio of the compound (III) to the sum total of the compounds (I), (II), (III) and (IV) ranges from 0.173 to 0.661, and

the weight ratio of the compound (IV) to the total sum of the compounds (I), (II), (III) and (IV) ranges from 0.001 to 0.077,

and that the mixture comprising the compounds (I), (II), (III) and (IV) has a Griffin's HLB value ranging from 5 to 12:

$$\begin{array}{l} CH_2-O(QO)_aA^1 \\ | \\ CH-O(QO)_bA^2 \\ | \\ CH_2-O(QO)_cA^3 \end{array} \qquad (I)$$

[wherein

| | |
|---|---|
| $A^1$, $A^2$ and $A^3$: | each an RCO group or a hydrogen atom with the proviso that one of $A^1$, $A^2$ and $A^3$ is an RCO group and the others thereof are each a hydrogen atom, wherein R represents a linear or branched alkyl or alkenyl group having 7 to 23 carbon atoms, |
| Q: | an alkylene group having 2 or 3 carbon atoms or an alkylene group mixture comprising one having two carbon atoms and one having three carbon atoms, and |
| a, b and c: | each a number of 0 or above, provided that the sum of a, b and c is 4 to 18 on the average], |

$$\begin{array}{l} CH_2-O(QO)_aB^1 \\ | \\ CH-O(QO)_bB^2 \\ | \\ CH_2-O(QO)_cB^3 \end{array} \qquad (II)$$

[wherein

| | |
|---|---|
| $B^1$, $B^2$ and $B^3$: | each an RCO group or a hydrogen atom with the proviso that two of $B^1$, $B^2$ and $B^3$ are each an RCO group and the other thereof is a hydrogen atom, wherein R group is as defined above, and |
| Q, a, b and c: | each as defined above], |

$$\begin{array}{l} CH_2-O(QO)_aD^1 \\ | \\ CH-O(QO)_bD^2 \\ | \\ CH_2-O(QO)_cD^3 \end{array} \qquad (III)$$

[wherein

| | |
|---|---|
| $D^1$, $D^2$ and $D^3$: | each an RCO group wherein R is as defined above, and |
| Q, a, b and c: | each as defined above], and |

$$\begin{array}{l} CH_2-O(QO)_aH \\ | \\ CH-O(QO)_bH \\ | \\ CH_2-O(QO)_cH \end{array} \qquad (IV)$$

[wherein

Q, a, b and c: each as defined above]

and

[softener base material (2)]

a mixture comprising the following components (A) and (B) wherein the weight ratio of the component (A) to the component (B) ranges from 2/1 to 1/9:

<component (A)>

a mixture comprising a compound represented by the following general formula (V) (hereinafter referred to as "compound (V)"), a compound represented by the following general formula (VI) (hereinafter referred to as "compound (VI)"), a compound represented by the following general formula (VII) (hereinafter referred to as "compound (VII)") and a compound represented by the following general formula (VIII) (hereinafter referred to as "compound (VII)"), provided that

the weight ratio of the compound (V) to the total sum of the compounds (V), (VI), (VII) and (VIII) ranges from 0.040 to 0.527,

the weight ratio of the compound (VI) to the total sum of the compounds (V), (VI), (VII) and (VIII) ranges from 0.133 to 0.469,

the weight ratio of the compound (VII) to the total sum of the compounds (V), (VI), (VII) and (VIII) ranges from 0.013 to 0.661, and

the weight ratio of the compound (VIII) to the total sum of the compounds (V), (VI), (VII) and (VIII) ranges from 0.001 to 0.417,

and that the mixture comprising the compounds (V), (VI) (VII) and (VIII) has a Griffind's HLB value ranging from 5 to 15, except the mixtures falling under the category of the softener base material (1):

$$\begin{array}{l} CH_2-O(QO)_xA^1 \\ | \\ CH-O(QO)_yA^2 \\ | \\ CH_2-O(QO)_zA^3 \end{array} \qquad (V)$$

[wherein

$A^1$, $A^2$, $A^3$ and Q: each as defined above, and

x, y and z: each a number of 0 or above, provided that the sum of x, y and z is 1 to 50 on the average],

$$\begin{array}{l} CH_2-O(QO)_xB^1 \\ | \\ CH-O(QO)_yB^2 \\ | \\ CH_2-O(QO)_zB^3 \end{array} \qquad (VI)$$

[wherein

$B^1$, $B^2$, $B^3$, Q, x, y and z: each as defined above] ,

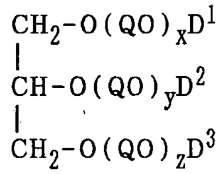

(VII)

[wherein

$D^1$, $D^2$, $D^3$, Q, x, y and z: each as defined above]

and

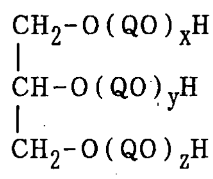

(VIII)

[wherein

Q, x, y and z: each as defined above], and

<component (B)>

at least one member selected from the following components (B-1) and

(B-2):

<<component (B-1)>>

a compound represented by the following general formula (X):

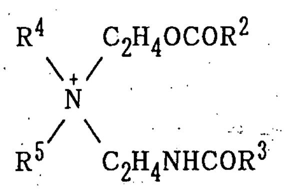

$X^-$ (X)

[wherein

$R^2$ and $R^3$: the same or different from each other a linear or branched alkyl or alkenyl group having 7 to 23 carbon atoms,
$R^4$ and $R^5$: each an alkyl group having 1 to 4 carbon atoms, and
$X^-$: a halide ion or $R^4SO_4$ wherein $R^4$ is as defined above], and

<<component (B-2)>>

a compound represented by the following general formula (XI):

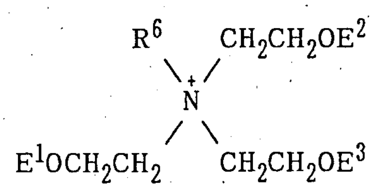

$Y^-$ (XI)

[wherein

$E^1$, $E^2$ and $E^3$: each an $R^7CO$ group or a hydrogen atom with the proviso that at least one of $E^1$, $E^2$ and $E^3$ is an $R^7CO$ group, wherein $R^7$ represents a linear or branched alkyl or alkenyl group having 7 to 23 carbon atoms,

$R^6$: an alkyl group having 1 to 4 carbon atoms, and

$Y^-$: a halide ion or $R^6SO_4$ wherein $R^6$ is as defined above].

Further, the present invention also provides a liquid softener composition comprising the above softener base material (1) and the component (B) described above as the constituent of the softener base material (2) wherein the weight ratio of the softener base material (1) to the component (B) ranges from 2/1 to 1/9.

The invention (2) relates to a liquid softener composition comprising the following component (2-A) and a component (2-b) represented by the formula (X) as described as component (B-1) in claim 1 wherein the weight ratio of the component (2-A) to the component (B-1) ranges from 2/1 to 1/9:

[component (2-A)]

a compound represented by the following general formula (2-I):

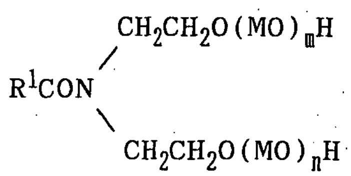

(2-I)

[wherein

$R^1$: a linear or branched alkyl or alkenyl group having 7 to 23 carbon atoms,

M: an alkylene group having 2 or 3 carbon atoms, and

m and n: each a number of 0 or above, provided that the sum of m and n is 0 to 4 on the average].

The invention (3) relates to a process for the preparation of a quaternary ammonium salt represented by the general formula (3-I):

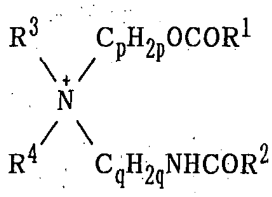

$X^-$ (3-I)

(wherein $R^1$ and $R^2$ represent the same or different from each other a linear or branched alkyl or alkenyl group having 7 to 35 carbon atoms; $R^3$ represents an alkyl or hydroxyalkyl group having 1 to 6 carbon atoms; $R^4$ represents an alkyl group having 1 or 2 carbon atoms; p and q are the same or different from each other an integer of 2 to 6; and X represents a halogen atom or an $R^4SO_4$ group) by the reaction of a tertiary amine corresponding to the quaternary ammonium salt with a quaternizing agent, characterized by conducting the reaction in the presence of a compound

represented by the general formula (3-II):

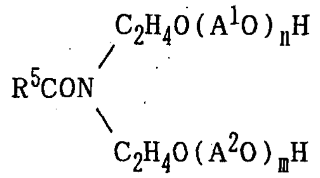

(3-II)

(wherein $R^5$ represents a linear or branched alkyl or alkenyl group having 7 to 35 carbon atoms; $A^1$ and $A^2$ represent the same or different from each other an alkylene group having 2 to 4 carbon atoms; and n and m each represent the average number of alkylene oxide molecules added with the proviso that the sum of n and m is 0 to 4, with a number "n" of $A^1$s or a number "m" of $A^2$s being the same or different from each other).

The invention (4) provides a process for the preparation of a quaternary ammonium salt with a polyhydric alcohol ester represented by the formula (4-I) which will be described below.

According to this process, the quaternary ammonium salts to be used in the inventions (1) and (2) can be prepared.

The present invention will now be described in more detail.

## Detailed Description of the Invention

[Softener base material (1)]

In the softener base material (1) comprising the compounds (I) to (IV), the mixing ratio must satisfy the requirement that the weight ratio of the compound (I) to the sum total of the compounds (I), (II), (III) and (IV) should range from 0.040 to 0.327, preferably 0.040 to 0.317, that of the compound (II) to the sum total of the compounds (I), (II), (III) and (IV) should range from 0.298 to 0.469, preferably 0.298 to 0.469, that of the compound (III) to the sum total of the compounds (I), (II), (III) and (IV) should range from 0.173 to 0.661, preferably 0.183 to 0.661 and that of the compound (IV) to the sum total of the compounds (I), (II), (III) and (IV) should range from 0.001 to 0.077, preferably 0.001 to 0.067.

When the mixing ratio satisfies the above requirement, the resulting softener composition will be goodin stability. Although the reason for this phenomenon is not always apparent, it is presumed that the softener base material (1) having such a mixing ratio as described above may have a lowered crystallinity and therefore the resulting liquid softener composition may be inhibited from the deposition of the base material (1) to cause neither thickening nor gelation nor any other appearance change.

In each of the general formulae (I), (II), (III) and (IV), the sum of a, b and c is 4 to 18, preferably 4 to 12 on the average. When the sum of a, b and c falls within said range it is especially possible to prepare a stable softener composition and to attain the desirable softening effect.

It is essential that the Griffin's HLB value of the softener base material (1) is 5 to 12, preferably 5.6 to 12. When the HLB value falls within said range, it is in particular possible to prepare a stable softener composition and to attain a satisfactory softening effect.

The Griffin's HLB value of a fatty acid ester of polyhydric alcohol is calculated by the following formula:

$$\text{Griffin's HLB} = 20 \times (1 - S/A)$$

[wherein

S: saponification value (mg KOH/g) of the fatty acid ester of polyhydric alcohol, and
A: acid value (mg KOH/g) of the fatty acid constituting the fatty acid ester of polyhydric alochol].

In the general formulae (I) to (III), R group is, e.g., at least one alkyl group selected from the group consisting of alkyl resulting from tallow fatty acid, that resulting from hardened tallow fatty acid, that of palm stearic acid and that of hardened palm stearic acid.

The softener base material (1) can be prepared by, e.g., the following synthesis processes (i) to (iv).

Synthesis process (i)

The softener base material (1) can be prepared by esterifying glycerol with a fatty acid and conducting the addition reaction of the obtained ester with ethylene oxide and/or propylene oxide. In the addition thereof with both ethylene oxide and propylene oxide, it is preferable that the addition reaction with propylene oxide be followed by that with ethylene oxide, though the addition reaction with either of ethylene oxide and propylene oxide may precede or the addition reactions with ethylene oxide and propylene oxide may be conducted simultaneously. Further, it is preferable that the number of propylene oxide molecules added to one glycerol skeleton be 3 or below.
Although the above esterification according to the synthesis process (i) can be conducted without a catalyst, it may be conducted in the presence of an acid catalyst such as sulfuric acid, hydrochloric acid or p-toluenesulfonic acid. Examples of the fatty acid to be used in the esterification include caprinic acid, lauric acid, myristic acid, palmitic acid, oleinic acid, stearic acid, isostearic acid, arachidic acid, behenic acid and fatty acids prepared from coconut oil, palm kernel oil, unhardened and hardened tallows, lard, palm oil, rape seed oil, fish oil, palm stearin and hardened palm stearin, which may be each used alone or as a mixture of two or more of them. Among these fatty acids, it is preferable to use coconut oil fatty acid, palm kernel oil fatty acid, palm oil fatty acid, unhardened or hardened tallow fatty acid, unhardened or hardened palm stearic acid or a mixture of two or more of them.
The addition reaction is conducted in the presence of NaOH, KOH, $NaOCH_3$, $KOCH_3$, an alkali metal salt of fatty acid or the like as catalyst. It is preferable that the amount of the catalyst to be added exceed the amount necessary for the addition reaction by the amount required for neutralizing the acid catalyst used for the above esterification.

Synthesis process (ii)

The softener base material (1) can be prepared by transesterifying a fatty acid ester with glycerol and conducting the addition reaction of the obtained ester with an alkylene oxide.
The catalyst to be used in the transesterification includes NaOH, KOH, $NaOCH_3$ and $KOCH_3$.
Examples of the fatty acid ester to be used in the synthesis process (ii) include esters of the fatty acids described in the section "Synthesis process (i)" with methanol, ethanol, propanol, butanol, ethylene glycol, glycerol, erythritol, pentaerythritol, xylitol, sorbitol and sorbitan.
Among these fatty acid esters, the following compounds are preferable:

$R\text{-}COOCH_3$, $R\text{-}COOCH_2CH_3$,

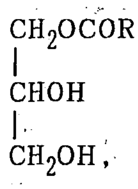 , 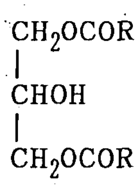 , 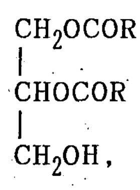 , 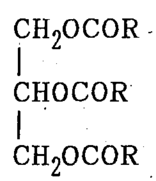

[wherein R is as defined above].
The addition reaction of the synthesis process (ii) is conducted in the presence of a catalyst such as NaOH, KOH, $NaOCH_3$, $KOCH_3$ or an alkali metal salt of fatty acid. The catalyst used in the preceding transesterification may be used as such in the addition reaction. The kind of alkylene oxide and the order of addition reaction are the same as described in the section "Synthesis process (i)".

Synthesis process (iii)

The softener base material (1) can be prepared by conducting the addition reaction of glycerol with an alkylene oxide and esterifying the obtained adduct with a fatty acid.
The addition reaction and esterification of the synthesis process (iii) may be conducted under the same conditions as those described in the section "Synthesis process (i)".
The fatty acid to be used in the esterification of the synthesis process (iii) may be selected from among those described in the section "Synthesis process (i)".

Synthesis process (iv)

The softener base material (1) can be prepared by conducting the addition reaction of glycerol with an alkylene oxide and transesterifying a fatty acid ester with the obtained adduct.

The addition reaction and transesterification of the synthesis process (iv) may be conducted under the same conditions as those described in the section "Synthesis process (ii)".

The fatty acid ester to be used in the transesterification of the synthesis process (iv) includes the following compounds: $R\text{-}COOCH_3$, $R\text{-}COOCH_2CH_3$

[wherein R is as defined above].

Specific examples of the softener base material (1) include adducts of mixtures comprising glycerides of tallow fatty acid and glycerol with alkylene oxides, those of mixtures comprising glycerides of partially hardened tallow fatty acid and glycerol with alkylene oxides, those of mixtures comprising glycerides of palm stearic acid and glycerol with alkylene oxides, those of mixtures comprising glycerides of hardened palm stearic acid and glycerol with alkylene oxides, those of mixtures comprising lauric acid esters and glycerol with alkylene oxides, those of mixtures comprising glycerides of palm kernel oil fatty acid and glycerol with alkylene oxides, those of mixtures comprising glycerides of coconut oil fatty acid and glycerol with alkylene oxides, and those of mixtures comprising glycerides of palm oil fatty acid and glycerol with alkylene oxides. These adducts of alkylene oxides are preferably those of ethylene oxide.

[Softener base material (2)]

The softener base material (2) according to the present invention is a mixture comprising the above components (A) and (B). The weight ratio of the component (A) to the component (B) ranges from 2/1 to 1/9, preferably 1/1 to 1/9, still preferably 1/2 to 1/9. When the weight ratio falls within the mentioned range it is in particular possible to attain the desired effects according to the present invention.

<Component (A)>

The component (A) according to the present invention is a mixture comprising the above compounds (V), (VI), (VII) and (VIII), except the mixtures falling under the category of the softener base material (1). In the component (A) comprising the compounds (V) to (VIII), the mixing ratio must satisfy the requirement that the weight ratio of the compound (V) to the sum total of the compounds (V), (VI), (VII) and (VIII) should range from 0.040 to 0.527, preferably 0.047 to 0.346, still preferably 0.163 to 0.346, that of the compound (VI) to the sum total of the compounds (V), (VI), (VII) and (VIII) should range from 0.133 to 0.469, preferably 0.310 to 0.458, still preferably 0.404 to 0.458, that of the compound (VII) to the sum total of the compounds (V), (VI), (VII) and (VIII) should range from 0.013 to 0.661, preferably 0.154 to 0.641, still preferably 0.154 to 0.375, and that of the compound (VIII) to the sum total of the compounds (V), (VI), (VII) and (VIII) should range from 0.001 to 0.417, preferably 0.002 to 0.096, still preferably 0.017 to 0.096.

When the mixing ratio satisfies the above requirement, the resulting softener composition will be good in stability. Although the reason for this phenomenon is not always apparent, it is presumed that the component (A) having such a mixing ratio as described above may have a lowered crystallinity and therefore the resulting liquid softener composition may be inhibited from the deposition of the component (A) to cause neither thickening nor gelation nor any other appearance change.

In each of the general formulae (V), (VI), (VII) and (VIII), the sum of x, y and z is 1 to 50, preferably 3 to 50, still preferably 3 to 40 on the average. When the sum of x, y and z falls within this range particularly a stable softener composition will be prepared and the desirable softening effect according to the present invention will be attained.

The component (A) constituting the softener base material (2) must have a Griffin's HLB value ranging from 5 to 15, so that a stable softener composition may be prepared and a satisfactory softening effect may be attained.

In the general formulae (V) to (VII), R group is, e.g., at least one alkyl group selected from the group consisting of alkyl resulting from coconut oil fatty acid, that resulting from palm kernel oil fatty acid, that resulting from palm oil fatty acid, that resulting from palm stearic acid, that resulting hardened palm stearic acid, that resulting from tallow fatty acid and that resulting from hardened tallow fatty acid.

The component (A) constituting the softener base material (2) can be prepared by the same processes as those for preparing the softener base material (1).

In the general formulae (V), (VI), (VII) and (VIII), the alkylene oxide added is preferably ethylene oxide.

<Component (B)>

The component (B) according to the present invention is at least one member selected from the group consisting of the compounds (B-1) represented by the general formula (X) and the compounds (B-2) represented by the general formula (XI).

<<Component (B-1)>>

In the general formula (X), $X^-$ represents a halide ion (such as $Cl^-$, $Br^-$ or the like), $CH_3SO_4^-$, $C_2H_5SO_4^-$, $C_3H_7SO_4^-$ or the like.

The quaternary ammonium salt (X) to be used as the component (B-1) can be prepared according, e.g., the following reaction formula:

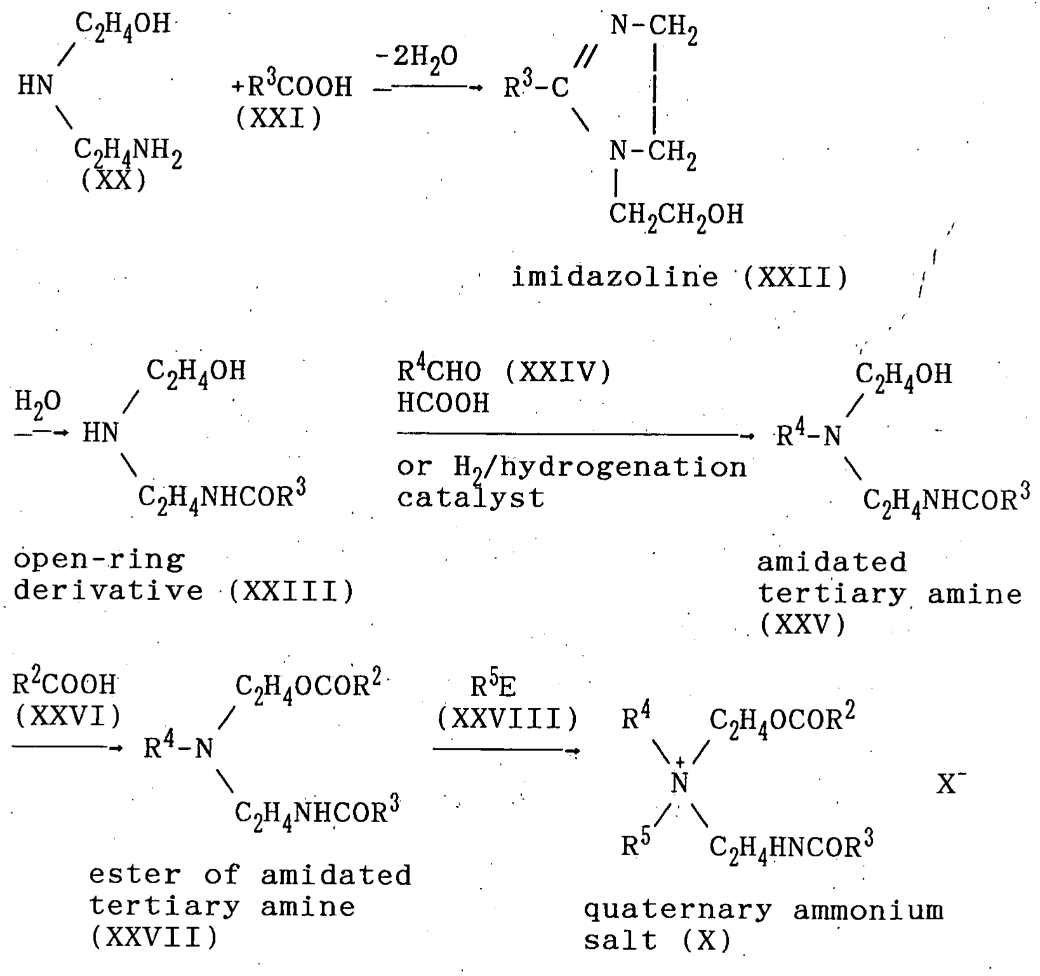


(XX)

(XXI)

imidazoline (XXII)

open-ring derivative (XXIII)

amidated tertiary amine (XXV)

ester of amidated tertiary amine (XXVII)

quaternary ammonium salt (X)

[wherein

$R^2$, $R^3$, $R^4$, $R^5$ and $X^-$: each as defined above, and
E: a halogen atom or $R^5$-$SO_4^-$ wherein $R^5$ is as defined above].

Specifically, an imidazoline (XXII) is first prepared by the reaction of N-(2-aminoethyl)ethanolamine (XX) with a fatty acid (XXI). The molar ratio of N-(2-aminoethyl)ethanolamine (XX) to the fatty acid (XXI) may range from 1.0 to 2.0, preferably 1.1 to 1.5. The reaction temperature may ranges from 150 to 250°C, preferably 180 to 230°C. It is preferable in order to enhance the dehydration efficiency that the reaction system be gradually vacuumized to about 5 Torr.

The fatty acid (XXI) to be used in the above reaction is generally one resulting from a natural fat or oil such as coconut oil, palm oil, tallow, rape seed oil or fish oil, or alternatively may be a synthetic fatty acid having 8 to 24 carbon atoms.

The imidazoline (XXII) thus prepared is hydrolyzed into an open-ring derivative (XXIII) in the presence of a catalyst such as NaOH or without a catalyst either in a mixed solvent such as water/ethanol or in water. In this hydrolysis, the amount of water used is 1 to 10 mol, preferably 2 to 5 mol per mol of the imidazoline (XXII), while ethanol is used in an amount of 0 to 50% by weight, preferably 10 to 30% by weight based on the sum total of the imidazoline (XXII) and water. Sodium hydroxide is used in an amount of 0 to 10% by mole, preferably 0 to 5% by mole based on the

imidazoline (XXII).

The open-ring derivative (XXIII) thus prepared is converted into an amidated tertiary amine (XXV) either by subjecting the derivative (XXIII) to the Leukart reaction with an aldehyde (XXIV) such as formaldehyde or acetaldehyde under conventional conditions or by alkylating the derivative (XXIII) through reduction (i.e., by reacting the derivative (XXIII) with an aldehyde (XXIV) and hydrogenating the obtained product in the presence of a hydrogenation catalyst). Then, the amidated tertiary amine (XXV) is esterified with a fatty acid (XXVI) into an ester of amidated tertiary amine (XXVII). The fatty acid (XXVI) to be used in this esterification is generally one resulting from a natural fat or oil such as coconut oil, palm oil, tallow, rape seed oil, fish oil or the like, or alternatively may be a synthetic fatty acid having 8 to 24 carbon atoms.

The ester of amidated tertiary amine (XXVII) thus prepared is quaternized with an quaternizing agent (XXVIII) in a conventional manner, followed by, if necessary, the replacement of the counter ion. Thus, a quaternary ammonium salt (X) is obtained. The quaternizing agent (XXVIII) usable in this case includes alkyl halides (such as methyl chloride, ethyl chloride and methyl bromide) and dialkylsulfuric acids (such as dimethylsulfuric acid and diethylsulfuric acid). The quaternization may be conducted in a solvent and examples of the solvent include lower alcohols such as ethanol, isopropanol and butanol, the softener base material (1) of the present invention, the component (A) of the softener base material (2) of the present invention and mixtures of two or more of them. The quaternary ammonium salt (X) thus prepared may be, if necessary, subjected to the replacement of the counter ion with an ion exchange resin or the like.

Examples of the component (B-1) are as follows:

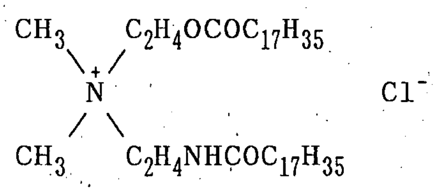

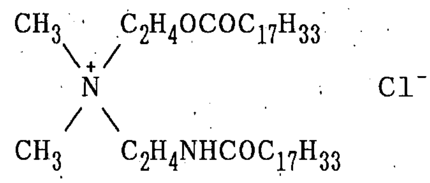

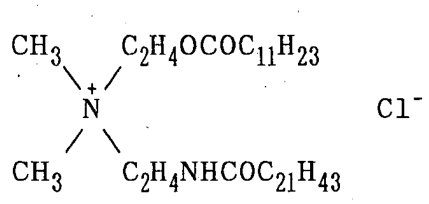

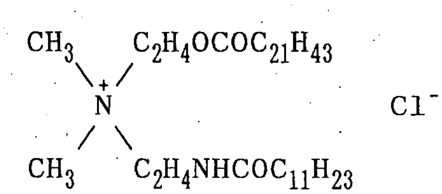

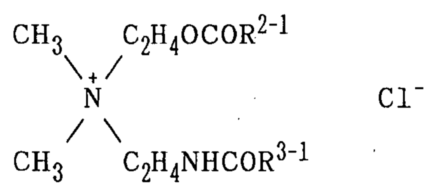

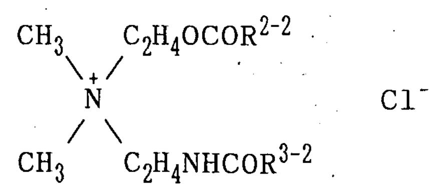

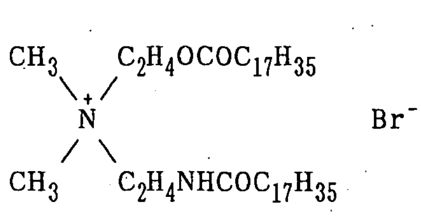

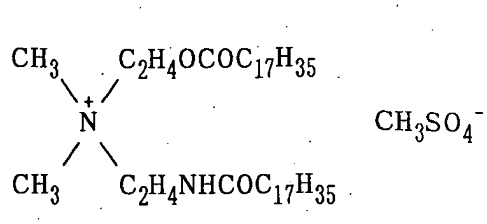

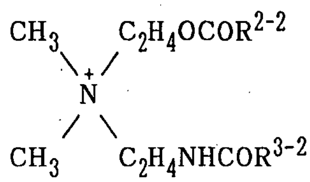

$CH_3SO_4^-$

[wherein

| | |
|---|---|
| $R^{2-1}$ and $R^{3-1}$: | each an alkyl group resulting from hardened tallow fatty acid, and |
| $R^{2-2}$ and $R^{3-2}$: | each an alkyl group resulting from hardened palm oil fatty acid] |

<Component (B-2)>

The component (B-2) according to the present invention is a compound represented by the general formula (XI).

In the general formula (XI) $Y^-$ is a halide ion (such as $Cl^-$, $Br^-$ or the like), $CH_3SO_4^-$, $C_2H_5SO_4^-$, $C_3H_7SO_4^-$ or the like.

Examples of the component (B-2) are as follows:

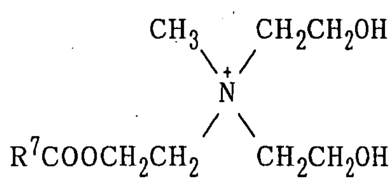

$CH_3SO_4^-$

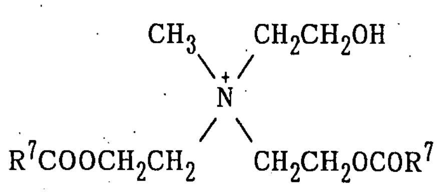

$CH_3SO_4^-$

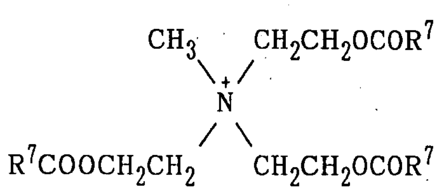

$CH_3SO_4^-$

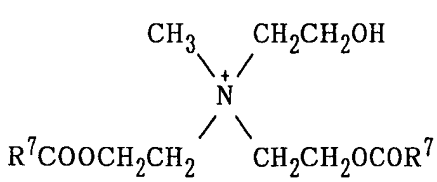

$Cl^-$

[wherein $R^7$: as defined above].

The component (B-2) can be prepared by, e.g., esterifying triethanolamine with a fatty acid and quaternizing the obtained ester. The solvent usable in this quaternization includes lower alcohols such as ethanol, isopropanol and butanol, the softener base material (1) of the present invention, the component (A) of the softener base material (2) of the present invention and mixture of two or more of them.

[Softener composition (1)]

The softener composition of the present invention comprises the above softener base material (1) or (2) and water.

Although the softener base material (1) can be used alone as a softener base material, the softener base material (1) may be used together with the component (B) of the softener base material (2). When the softener base material (1) is used together with the component (B) of the softener base material (2), it is desirable that the weight ratio of the softener base material (1) to the component (B) of the softener base material (2) ranges from 2/1 to 1/9, more desirably 1/1 to 1/9, most desirably 1/2 to 1/9. When the weight ratio lies within this range, a particularly excellent softening effect can be attained.

According to the present invention, the softener base material (1) or (2) or the softener base materials (1) and (2) (hereinafter referred to as "the softener base material of the present invention) is used in an amount of 3 to 40% by weight, preferably 5 to 30% by weight, still preferably 5 to 25% by weight based on the softener composition.

When the amount of the softener base material of the present invention lies within this range, a particularly desirable softening effect according to the present invention can be attained, and

the resulting softener composition will not have too high a viscosity to be easily taken out of a bottle unfavorably.

Among the softener compositions according to the present invention, one containing the softener base material of the present invention in a larger amount tends to thicken during storage. In order to prevent such thickening, the softener composition of the present invention may further contain, as component (C), a polyether compound prepared by the addition reaction of a compound having at least three active hydrogen atoms with an alkylene oxide having 2 or 3 carbon atoms and having an average molecular weight of 5,000 to 2,000,000 (wherein the total amount of ethylene oxide moieties is 55% by weight or above based on the component (C)), or a derivative thereof.

Examples of the compound having at least three active hydrogen atoms to be used as the starting material for preparing the component (C) include polyhydric alcohols such as trimethylolpropane, diethanolamine, triethanolamine, glycerol, pentaerythritol, sorbitol, sucrose, polyglycerol, polyvinyl alcohol and partially saponified polyvinyl acetate; polyhydric phenols such as phenolic resins and alkylphenol-formalin condensates; and polyamine compounds such as polyethyleneimines (e.g., ethylenediamine, diethylenetriamine, triethylenetetramine, tetraethylenepentamine and pentaethylenehexamine). Further, partially amidated derivatives and N-alkylated derivatives of these polyamine compounds can be used, as far as they have at least three active hydrogen atoms.

The component (C) can be prepared by conducting the addition reaction of a compound having at least three active hydrogen atoms with an alkylene oxide component containing ethylene oxide as an essential component in a conventional manner. In particular, the component (C) is preferably an adduct of the compound with ethylene oxide alone or an adduct thereof with both ethylene oxide and propylene oxide wherein the whole or part of the ethylene oxide and propylene oxide molecules are arranged in block. Although the order of the addition reactions may be arbitrary, an adduct prepared by conducting the addition reaction of a compound having at least three active hydrogen atoms with propylene oxide (hereinafter abbreviated to "PO") and thereafter the addition reaction of the obtained adduct with ethylene oxide (hereinafter abbreviated to "EO") is preferable, because a softener composition comprising such an adduct is inhibited from thickening during storage even when the content of the base material of the present invention is high.

The molecular weight of the component (C) may range from 5,000 to 2,000,000, preferably 10,000 to 100,000. Further, the total weight of oxyethylene moieties (EO chains) accounts for at least 55% by weight, preferably at least 80% by weight of the molecular weight of the component (C).

When the molecular weight of the component (C) is less than 5,000, the resulting softener composition will not be sufficiently inhibited from thickening during storage, while when it exceeds 2,000,000, the resulting softener composition will have too high a viscosity to be easily taken out of a bottle unfavorably.

When the total weight of the oxyethylene moieties accounts for less than 55% by weight of the molecular weight of the component (C), the resulting softener composition will not sufficiently be inhibited from thickening during storage.

The derivative of the polyether compound usable as the component (C) includes products of crosslinking of the polyether compound with isocyanate compounds; products prepared by sulfonating, phosphorylating and carboxyalkylating the polyether compound at the terminal hydroxy group; products prepared by esterifying the terminal hydroxy group of the polyether compound with fatty acids; and cationic products prepared by converting a part of the nitrogen

atoms of the polyether compound into cations. Among these derivatives, products prepared by esterifying the terminal hydroxy group of the polyether compound with fatty acids and cationic products prepared by converting a part of the nitrogen atoms of the polyether compound into cations are particularly preferable.

The fatty acid to be used in the above esterification is preferably one having 7 to 23 carbon atoms. The number of double bonds of the fatty acid or whether the fatty acid has a branch or not has not any great influence on the performance.

The cationic products include products prepared by converting the polyether compound into cations with dialkylsulfuric acids and alkyl halides and those prepared by neutralizing it with acetic acid and alkylbenzenesulfonic acid.

In order to attain both the desirable softening performance according to the present invention and the inhibition of the softener composition from thickening during storage simultaneously, it is desirable that the amount of the component (C) is so adjusted that the amount of the component (C) is 0.5 to 5% by weight, preferably 1 to 3% by weight based on the softener composition, that the weight ratio of the component (C) to the softener base material of the present invention is 1/100 to 1/2.5, preferably 1/50 to 1/5, and that the total amount of the softener base material of the present invention and the component (C) is 3.5 to 45% by weight or 4 to 50% by weight, preferably 11 to 40% by weight, still preferably 14 to 35% by weight based on the softener composition.

The softener composition of the present invention may further contain a linear or branched, saturated or unsaturated fatty acid having 8 to 24 carbon atoms as component (D) to improve the softening performance and storage stability of the composition. It is preferable that the weight ratio of the component (D) to the softener base material of the present invention range from 0.5/100 to 1/1, still preferably 1/100 to 1/2. Further, it is preferable that the total amount of the softener base material of the present invention and the component (D) be 4 to 50% by weight based on the softerner composition. Examples of the fatty acid to be used here include caprinic acid, lauric acid, myristic acid, palmitic acid, oleic acid, stearic acid, isostearic acid, arachidic acid, behenic acid, fatty acids prepared from coconut oil, palm kernel oil, unhardened and hardened tallows, lard, palm oil, rape seed oil, fish oil, palm stearin and hardened palm stearin, and mixtures of two or more of them.

Further, the softener composition of the present invention may contain a softener base material represented by the following general formula (XXX-1), (XXX-2) or (XXX-3) and the amount of this softerner base material is 0 to 15% by weight based on the composition:

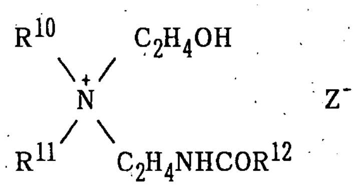 (XXX-1)

[wherein

$R^{10}$ and $R^{11}$: each an alkyl group having 1 to 4 carbon atoms,
$R^{12}$: a linear or branched alkyl or alkenyl group having 7 to 23 carbon atoms, and
$Z^-$: a halide ion or an $R^8SO_4^-$ group wherein $R^8$ represents an alkyl group having 1 to 4 carbon atoms and $Z^-$ and $X^-$ may be the same or different from each other],

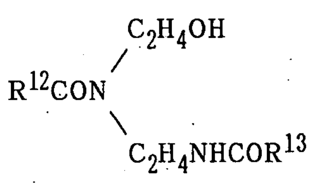 (XXX-2)

[wherein
$R^{12}$: as defined above, and
$R^{13}$: a linear or branched alkyl or alkenyl group having 7 to 23 carbon atoms], and

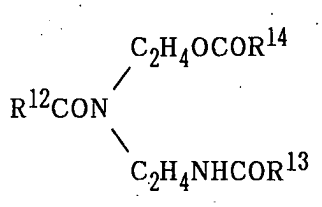

(XXX-3)

[wherein

$R^{12}$ and $R^{13}$: each as defined above, and
$R^{14}$: a linear or branched alkyl or alkenyl group having 7 to 23 carbon atoms].

Further, the softener composition of the present invention may further contain a conventional softener, so far as the object of the present invention is not marred.

The softener composition of the present invention may further contain an electrolyte such as NaCl, $CaCl_2$, $MgCl_2$ or N-methyl-N,N,N-triethanolammonium salt to control the viscosity of the composition. The amount of the electrolyte used is 0 to 5% by weight, preferably 0.01 to 2% by weight based on the composition.

The softener composition of the present invention may further contain an acid or an alkali to regulate the pH of the composition. It is desirable from the standpoints of the viscosity and storage stability of the composition that the pH of the composition be regulated within the range of 1.5 to 6.5.

Although the softener composition of the present invention is highly stable to long-term storage, the softener composition may further contain a nonionic surfactant such as polyoxyethylene(5-50 mole) alkyl or alkenyl($C_{12}$-$C_{24}$) ether or polyoxyethylene(5-50 mole) alkyl or alkenyl($C_{12}$-$C_{24}$) amine ; a hydrotrop or hydrotropic agent or dispersing agent such as ethylene glycol, propylene glycol, glycerol or urea; or a lower alcohol such as ethanol or isopropyl alcohol for the purpose of enhancing the stability of the composition under severer storage conditions. Further, the composition of the present invention may further contain a pigment or a dye for the purpose of improving the appearance of the composition, a silicone for the purpose of improving the antifoaming properties in rinsing, and/or a perfume for the purpose of improving the comfortableness in using or that of the treated clothes.

The softener composition of the present invention comprises the above essential and optional components and the balance of water.

A process for preparing the softener composition of the present invention will now be described, though the present invention is not limited to this process. Namely, a mixture comprising the softener base material of the present invention and optional components (exclusive of the component (C)) is molten and gradually dropped into ion-exchanged water kept at 60°C by heating under stirring to form an emulsion, followed by if necessary, the addition of the component (C). A nonionic surfactant may be preliminarily added to the ion-exchanged water and an inorganic salt may be added after the addition of the softener base material of the present invention and optional components to control the viscosity of the composition.

The invention (2) will now be described in detail.

[Component (2-A)]

A compound represented by the above general formula (2-I) in the invention is used as the component (2-A) .

In the general formula (2-I), the sum of m and n is 0 to 4 on the average. When the sum of m and n exceeds 4, no high softening effect will be attained. It is particularly preferable that both m and n be each 0.

When a fatty acid monoethanolamide or an adduct thereof with ethylene oxide which has a chemical structure similar to that of the component (2-A) is used instead of the component (2-A), the resulting composition will be poor in storage stability.

In the general formula (2-I), $R^1$ is, e.g., at least one alkyl group selected from the group consisting of alkyl group resulting from coconut oil fatty acid, alkyl group resulting from palm kernel oil fatty acid, alkyl resulting from palm oil fatty acid, alkyl group resulting from palm stearic acid, alkyl group resulting from hardened palm stearic acid, alkyl group resulting from tallow fatty acid and alkyl group resulting from hardened tallow fatty acid.

Examples of the component (2-A) are as follows:

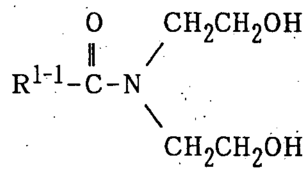

[wherein $R^{1-1}$: an alkyl group resulting from tallow fatty acid]

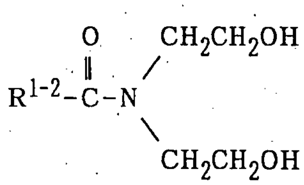

[wherein $R^{1-2}$: an alkyl group resulting from hardened tallow fatty acid]

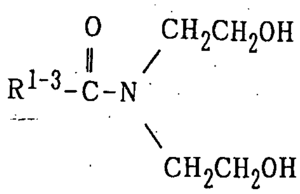

[wherein $R^{1-3}$: an alkyl group resulting from palm stearic acid]

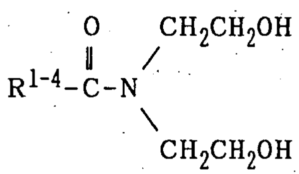

[wherein $R^{1-4}$: an alkyl group resulting from hardened palm stearic acid]

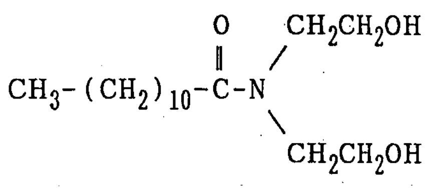

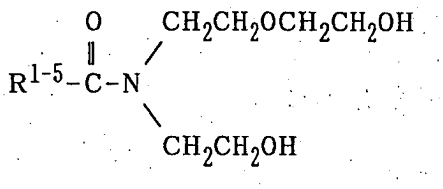

[wherein $R^{1-5}$: an alkyl group resulting from coconut oil fatty acid]

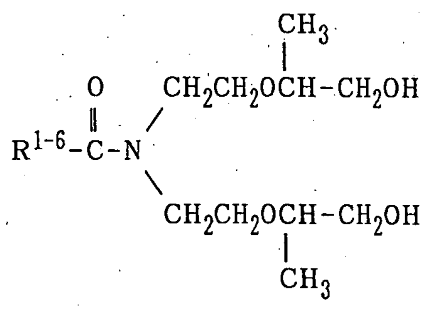

[wherein $R^{1-6}$: an alkyl group resulting from palm kernel oil fatty acid]

[Softener composition (2)]

The composition of the invention comprises the components (2-A) and (2-B).

The weight ratio of the component (2-A) to the component (2-B) may range from 2/1 to 1/9, preferably 1/1 to 1/9, still preferably 1/2 to 1/9. When the weight ratio falls within the range, particularly the desired softening effect can be attained and the clothes treated with the resultinq composition will not be greasy to the touch, which is desirable for the user.

The total amount of the components (2-A) and (2-B) is 3 to 40% by weight, preferably 5 to 30% by weight, still preferably 5 to 25% by weight based on the composition. When the total amount of the components (2-A) and (2-B) lies within the range, particularly the desired softening effect can be attained and the resulting composition will not have too high a viscosity.

The composition of the invention (2) may further contain the components (C) and/or the other optional additives described for the invention (1).

In the invention (3), the compound represented by the general formula (3-II) is particularly preferably one wherein $R^5CO$ is a fatty acid residue resulting from a natural fat or oil and n and m are each 0.

The compound represented by the general formula (3-II) to be used in the invention can be prepared, for example, as follows.

Namely, the compound represented by the general formula (3-II) can be prepared by reacting a natural fat or oil with diethanolamine in the presence of a base catalyst to form an amide, and, if necessary, gradually adding an alkylene oxide to conduct addition reaction. The natural fat or oil can be replaced by a lower alcohol ester of fatty acid. When a natural fat or oil is used as the starting material, the product represented by the general formula (3-II) also contains glycerol or an adduct thereof with alkylene oxide as by-product, but can be used as such in the invention without purification.

The natural fat or oil usable in the above reaction includes tallow, palm oil, palm stearin oil, palm kernel oil, coconut oil, products of partial and complete hardening of these oils and fats and mixture of two or more of them. Examples of the lower alcohol ester of fatty acid usable in the reaction include methyl and ethyl esters of fatty acids having 8 to 36 carbon atoms such as octanoic acid, lauric acid myristic acid, palmitic acid, stearic acid and oleic acid. The alkylene oxide usable in the reaction includes ethylene oxide, propylene oxide, butylene oxide and mixtures of two or more of them, among which ethylene oxide is preferable. When a mixture of alkylene oxides is used, the oxides may be reacted simultaneously or successively. The base catalyst usable therein includes potassium hydroxide, sodium hydroxide and sodium methylate.

The amidation of a natural fat or oil or a lower alcohol ester of fatty acid with diethanolamine is conducted at a temperature ranging from 60 to 200°C, preferably 80 to 150°C, while the addition reaction with an alkylene oxide is conducted at a temperature ranging from 100 to 200°C, preferably 140 to 170°C.

In the above reaction of a natural fat or oil or a lower alcohol ester of fatty acid with diethanolamine, diethanolamine is used in an amount of 0.8 to 1.2 mol, preferably 0.9 to 1.1 mol per mol of the ester group of the natural fat or oil or lower alochol ester. The number of alkylene oxide molecules added to one diethanolamide molecule is 0 to 4, preferably 0 to 3.

The quaternization for preparing a quaternary ammonium salt represented by the general formula (3-I) is conducted by mixing a compound represented by the general formula (3-II) with a tertiary amine represented by the following general formula (3-III) corresponding to the quaternary ammonium salt represented by the general formula (3-I) and adding a quaternizing agent to the obtained mixture:

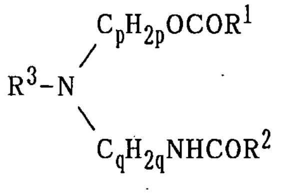

(3-III)

(wherein $R^1$, $R^2$, $R^3$, p and q are each as defined above).

The compound represented by the general formula (3-II) is mixed with the tertiary amine represented by the general formula (3-III) at a weight ratio of (3-II) to (3-III) of 90/10 to 1/99, preferably 50/50 to 5/95. An alcoholic solvent such as isopropyl alcohol or ethanol may be added in an amount of at most 20% by weight based on the tertiary amine at need, though the addition thereof in an amount exceeding 20% by weight is undesirable for the object of the invention. The quaternizing agent includes chloromethane, bromomethane iodomethane, dimethylsulfuric acid and diethylsulfuric acid. The quaternization is conducted at a temperature ranging from 60 to 140°C, preferably 80 to 120°C.

Among the thus-prepared quaternary ammonium salts represented by the general formula (3-I), one wherein $R^1$CO- group and $R^2$CO- group are each a fatty acid residue resulting from a natural fat or oil, $R^3$ and $R^4$ are each a methyl group, p is 2, q is 2 or 3, and X is Cl is particularly preferable.

Among the tertiary amines represented by the general formula (3-III) useful as the starting material of the quaternization, one wherein p and q are each 2 can be prepared by, e.g., the following reaction formula:

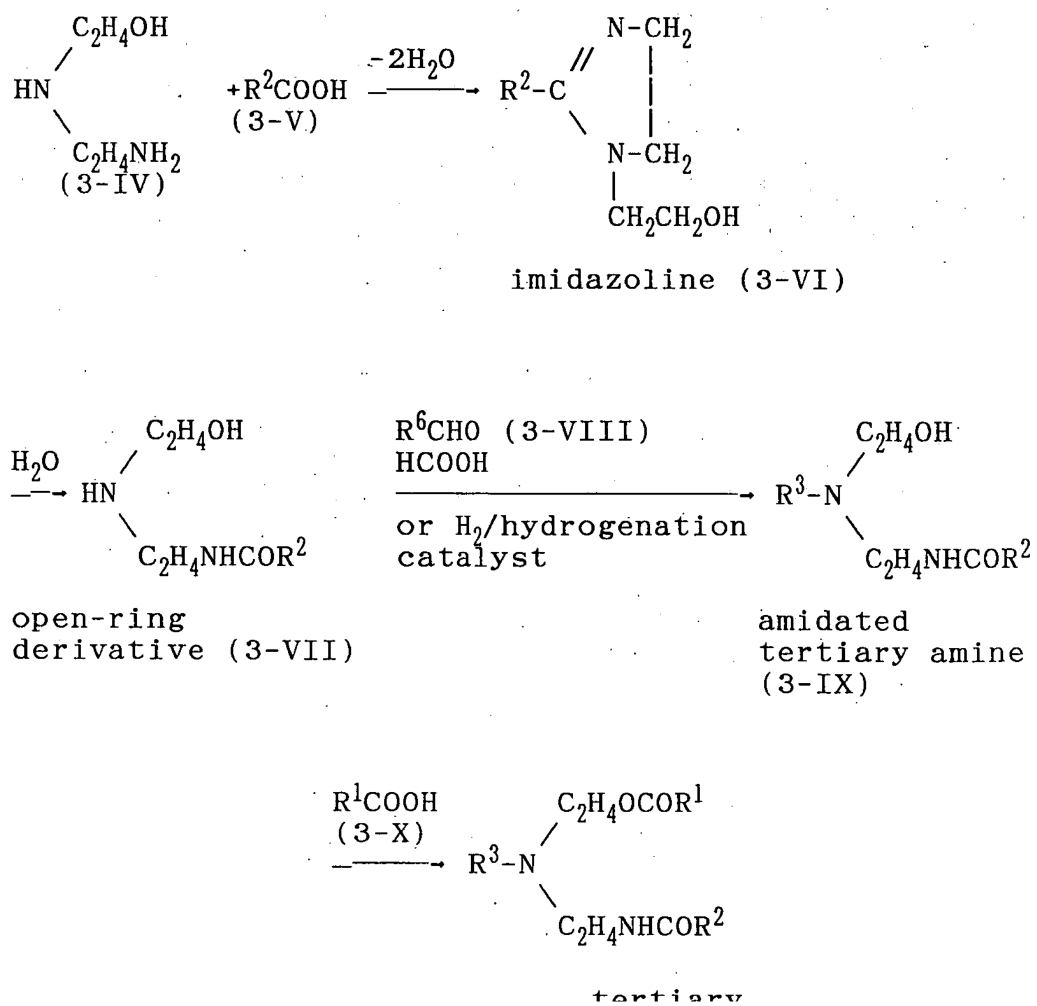


(3-IV)

imidazoline (3-VI)

open-ring derivative (3-VII)

amidated tertiary amine (3-IX)

tertiary

tertiary amine (3-III-1)

(wherein $R^1$, $R^2$ and $R^3$ are each as defined above; and $R^6$ represents H or an alkyl or hydroxyalkyl group having 1 to 3 carbon atoms)

Namely, an imidazoline (3-VI) is first prepared by reacting N-(2-aminoethyl)ethanolamine (3-IV) with a fatty acid (3-V). In this reaction, the molar ratio of N-(2-aminoethyl)ethanolamine (3-IV) to the fatty acid (3-V) ranges from 1.0 to 2.0, preferably 1.1 to 1.5 and the reaction temperature ranges from 150 to 250°C, preferably 180 to 230°C. It is desirable that the pressure of the reaction system is gradually reduced from normal pressure to about 5 Torr to enhance the dehydration efficiency.

The fatty acid (3-V) to be used in the above reaction is generally one resulting from a natural fat or oil such as coconut oil, palm oil, tallow, rape seed oil, fish oil or a product of partial or complete hardeneing of such an oil or fat, or alternatively may be a synthetic fatty acid having 8 to 36 carbon atoms.

The imidazoline (3-VI) thus prepared is hydrolyzed into an open-ring derivative (3-VII) either in a mixed solvent such as water/ethanol or in water either in the presence of a catalyst such as NaOH or without a catalyst. In this hydrolysis, water is used in an amount of 1 to 10 mol, preferably 2 to 5 mol per mol of the imidazoline (3-VI), while ethanol is used in an amount of 0 to 50% by weight, preferably 10 to 30% by weight based on the total weight of the imidazoline (3-VI) and water. Sodium hydroxide is used in an amount of 0 to 10% by mole, preferably 0 to 5% by mole based on the imidazoline (3-VI).

The open-ring derivative (3-VII) thus prepared is converted into an amidated tertiary amine (3-IX). either by subjecting the derivative (3-VII) to the Leukart reaction with an aldehyde (3-VIII) such as formaldehyde or acetaldehyde under conventional conditions or by alkylating the derivative (3-VII) through hydrogenation (i.e., by reacting the derivative (3-VII) with an aldehyde (3-VIII) and hydrogenating the obtained product in the presence of a hydrogenation catalyst). Then, the amidated tertiary amine (3-IX) is esterified into a tertiary amine (3-III-1) with a fatty acid (3-X). The fatty acid (3-X) to be used in this esterification is generally one resulting from a natural fat or oil such as coconut oil, palm oil, tallow, rape seed oil, fish oil or a product of partial or complete hardening of such an oil or fat, or alternatively may be a synthetic fatty acid having 8 to 36 carbon atoms.

Among the tertiary amines represented by the general formula (3-III) useful as the starting material of the quaternization, one wherein p is 2 and q is 3 can be prepared by, e.g., the following reaction formula:

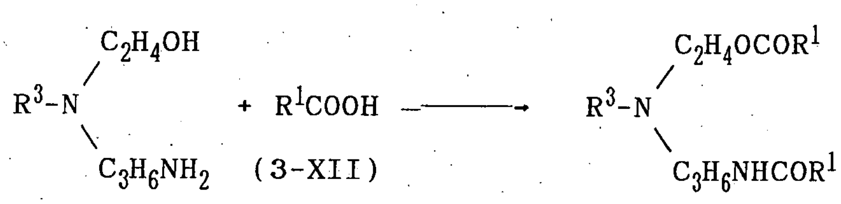

(3-XII)

(3-XI)

tertiary amine (3-III-2)

(wherein $R^1$ and $R^3$ are each as defined above, and the two $R^1$s in the formula (3-III-2) may be the same or different from each other).

Namely, a tertiary amine (3-III-2) can be prepared by reacting a compound (3-XI) prepared by cyanoethylating an N-(lower alkyl)ethanolamine or diethanolamine and hydrogenating the obtained product with a fatty acid (3-XII) having 8 to 36 carbon atoms. The fatty acid (3-XII) to be used in the reaction is generally one resulting from a natural fat or oil such as coconut oil, palm oil, tallow, rape seed oil, fish oil or a product of partial or complete hardening of such an oil or fat, or alternatively may be a synthetic fatty acid having 8 to 36 carbon atoms. These fatty acids may be each used alone or as a mixture of two or more of them.

The invention (4) provides a process for the preparation of a quaternary ammonium salt which comprises reacting a tertiary amine with a quaternizing agent, characterized by conducting the reaction in the presence of a polyhydric alcohol ester represented by the following general formula (4-I):

$$G[-OH]_p[-(OA)_mOH]_q[-O\overset{O}{\overset{\|}{C}}R]_r[-(OA)_nO\overset{O}{\overset{\|}{C}}R]_s \qquad (4\text{-}I)$$

[wherein

G group: a residue defined by removing all alcoholic hydroxyl groups from a polyhydric alcohol, [-OR] group, [-$(OA)_m$OH] group,

$$[-O\overset{O}{\overset{\|}{C}}R]$$

group and

$$[-(OA)_nO\overset{O}{\overset{\|}{C}}R]$$

group:
each a group bonded to the G group at a removed alcoholic hydroxyl group,

wherein A represents an alkylene group having 2 to 4 carbon atoms; R represents a linear or branched alkyl or alkenyl group having 7 to 23 carbon atoms; and m and n are each a number of 0 to 100, with a number "m plus n" of As being the same or different from each other, and

p, q, r and s: each a number of 0 or above wherein the sum of p, q, r and s represents the total number of alcoholic hydroxyl groups of the starting polyhydric alcohol, provided that the sum of p and q or that of r and s must not be 0].

The polyhydric alcohol ester represented by the above general formula (4-I) to be used in the invention is preferably at least one member selected from the group consisting of the following compounds (a), (b) and (c):

(a) fatty acid esters of penataerythritol (having at least one hydroxyl group) and adducts of these esters with alkylene oxides (wherein the alkylene oxide has 2 or 3 carbon atoms),
(b) fatty acid esters of glycerol (having at least one hydroxyl group) and adducts of these esters with alkylene oxides (wherein the alkylene oxide has 2 or 3 carbon atoms), and
(c) fatty acid esters of sorbitan (having at least one hydroxyl group) and adducts of these esters with alkylene oxides (wherein the alkylene oxide has 2 or 3 carbon atoms).

Among these polyhydric alcohol esters, the fatty acid esters of glycerol (having at least one hydroxyl group) and adducts of the esters with alkylene oxide (wherein the alkylene oxide has 2 or 3 carbon atoms) described in the above item (b) are preferable, with products of the reaction of natural fats and oils with glycerol and alkylene oxides (at a molar ratio of the fat or oil to glycerol to the alkylene oxide of 1 : (0.1 to 5) : (2 to 100) being still preferable.
The polyhydric alcohol ester represented by the above general formula (4-I) to be used in the invention can be prepared by, e.g., the following known processes (4-i) to (4-vi).

Preparation process (4-i)

$$G[-OH]_{p+q+r+s} + (r'+s')RCOOH \xrightarrow{-(r'+s')H_2O} \text{polyhydric alcohol ester} \quad (4\text{-}I\text{-}1)$$

polyhydric alcohol — fatty acid

[wherein

G, R, p, q, r and s: each as defined above, and
r' and s': each a number satisfying the relationship:

$$0 < (r' + s') < (p + q + r + s)]$$

According to the preparation process (4-i), a polyhydric alcohol is esterified with a fatty acid. The molar ratio of the polyhydric alcohol to the fatty acid is selected in such a range that the resulting polyhydric alcohol ester (4-I-1) has a hydroxyl group.

This esterification may be conducted in the presence of an acid catalyst such as sulfuric acid, hydrochloric acid or p-toluenesulfonic acid, though it can be conducted without a catalyst.

Examples of the polyhydric alcohol to be used in this process include glycerol, erythritol, pentaerythritol, sorbitol and sorbitan, which may be each used alone or as a mixture of two or more of them.

Examples of the fatty acid to be used in this process include caprinic acid, lauric acid, myristic acid, palmitic acid, oleic acid, stearic acid, isostearic acid, arachidic acid, behenic acid and fatty acids prepared from unhardened and hardened animal fats (such as tallow and lard), palm oil, rape seed oil and fish oil, which are each used alone or as a mixture of two or more of them.

Preparation process (4-ii)

$$G[-OH]_{p+q+r+s} + (r'+s')RCOOR' \xrightarrow{-(r'+s')R'OH} \text{polyhydric alcohol ester} \quad (4\text{-}I\text{-}1)$$

polyhydric alcohol — fatty acid ester

[wherein

G, R, p, q, r, s, r' and s': each as defined above,

and

R': a residue defined by removing a mono- or polyhydric alcohol hydroxyl group(s)]

According to the preparation process (4-ii), a fatty acid ester is transesterified with a polyhydric alcohol. This transesterification is conducted in the presence of a catalyst such as NaOH, KOH, $NaOCH_3$, $KOCH_3$ or the like.

Examples of the fatty acid ester to be used in this process include esters of the fatty acids described for the preparation process (4-i) with methanol, ethanol, propanol, butanol, ethylene glycol, glycerol, erythritol, pentaerythritol, xylitol, sorbitol and sorbitan; and natural fats and oils.

Preparation process (4-iii)

polyhydric alcohol ester (4-I-1) + (n+m) mol of $C_2$-$C_4$ alkylene oxide

$\longrightarrow$ polyhydric alcohol ester (4-I-2)

[wherein

| | |
|---|---|
| polyhydric alcohol ester (4-I-1): | one prepared by the preparation process (4-i) or (4-ii), and |
| m and n: | each as defined above]. |

According to the preparation process (4-iii), a polyhydric alcohol ester (4-I-2) can be prepared by the addition reaction of the polyhydric alcohol ester (4-I-1) prepared by the preparation process (4-i) or (4-ii) with an alkylene oxide having 2 to 4 carbon atoms. This addition reaction is conducted in the presence of a catalyst such as NaOH, KOH, $NaOCH_3$, $KOCH_3$, an alkali metal salt of fatty acid or the like.

In the addition reaction of alkylene oxide, it is preferable that the molar ratio of the alkylene oxide to the polyhydric alcohol ester (4-I-1) range from 1/1 to 100/1, still preferably 1/1 to 50/1.

Preparation process (4-iv)

polyhydric alcohol ester (4-I-2) + (r"+s")RCOOH or RCOOR'

$\xrightarrow[-(r"+s")R'OH]{-(r"+s")H_2O \text{ or}}$ polyhydric alcohol ester (4-I-3)

[wherein

| | |
|---|---|
| R and R': | each as defined above, |
| polyhydric alcohol ester (4-I-2): | polyhydric alcohol ester (4-I-2) prepared by the preparation process (4-iii), and |
| r" and s": | each a number satisfying the relationship: 0 < (r'+ s'+ r"+ s") < (p + q + r + s), wherein r', s', p, q, r and s are each as defined above] |

According to the preparation process (4-iv), the polyhydric alcohol ester (4-I-2) prepared by the preparation process (4-iii) is reacted with a fatty acid or an ester thereof selected from among those described for the preparation processes (4-i) and (4-ii) under the same conditions as those described for the preparation processes (4-i) and (4-ii).

Although the polyhydric alcohol ester (4-I-3) thus prepared contains unreacted polyhydric alcohol ester or unreacted fatty acid, such contamination is acceptable so far as the effect of the present invention is not marred.

Preparation process (4-v)

$$G[-OH]_{p+q+r+s} + (r'+s')RCOOR' + (m+n) \text{ mol of } C_2\text{-}C_4 \text{ alkylene oxide}$$

polyhydric alcohol — fatty acid ester

$$\longrightarrow \text{polyhydric alcohol ester (4-I-2)}$$

[wherein G, R, R', p, q, r, s, r', s', m and n are each as defined above].

In the preparation process (4-v), the molar ratio of the polyhydric alcohol to the fatty acid ester is selected in such a range that the resulting polyhydric alcohol ester (4-I-2) has a hydroxyl group. It is preferable that the molar ratio of the alkylene oxide to the polyhydric alcohol range from 1/1 to 100/1, still preferably 5/1 to 50/1.

The catalyst to be used in this process may be selected from among those described for the preparation process (4-iii).

Preparation process (4-vi)

$$G[-OH]_{p+q+r+s} + (m+n) \text{ mol of } C_2\text{-}C_4 \text{ alkylene oxide} \longrightarrow$$

polyhydric alcohol

$$\text{adduct of polyhydric alcohol with alkylene oxice} \xrightarrow[-(r'+s')H_2O]{(r'+s')RCOOH}$$

polyhydric alcohol ester (4-I-2)

[wherein G, R, p, q, r, s, r', s', m and n are each as defined above]

According to the preparation process (4-vi), the addition reaction of a polyhydric alcohol with an alkylene oxide is conducted (under the same conditions as those described for the preparation process (4-iii)), followed by the esterification of the obtained adduct with a fatty acid (under the same conditions as those described for the preparation process (4-i)).

Examples of the polyhydric alcohol ester (4-I) prepared by the above preparation processes (4-i) to (4-vi) include the following compounds, which may be each used alone or as a mixture of two or more of them:

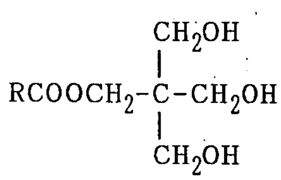
,
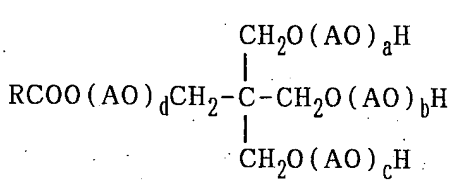

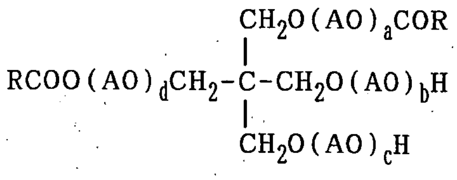 , 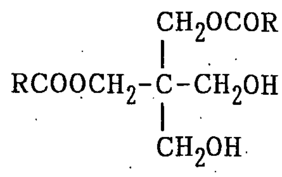

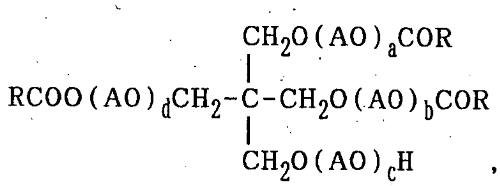 , 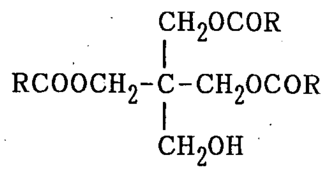

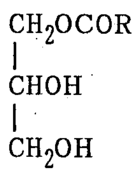 , 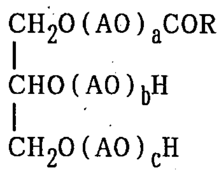 , 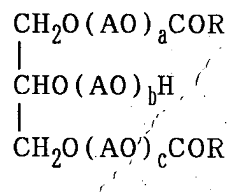

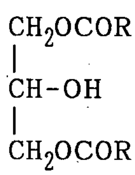 , 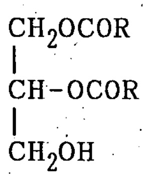 , 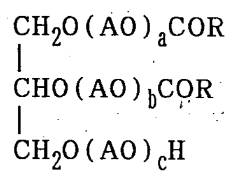

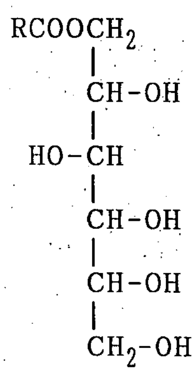 , 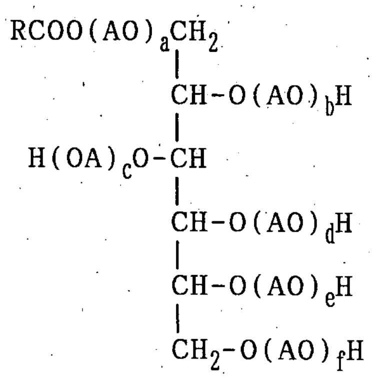

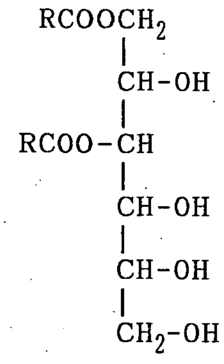 , 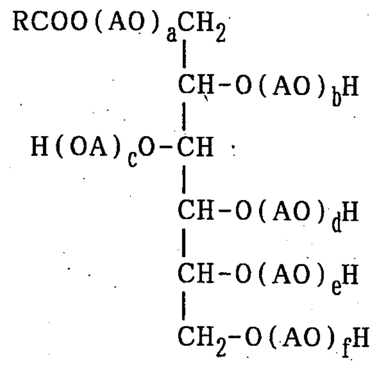

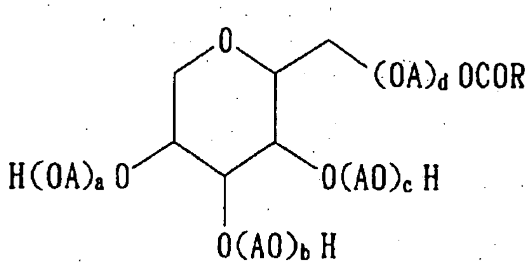

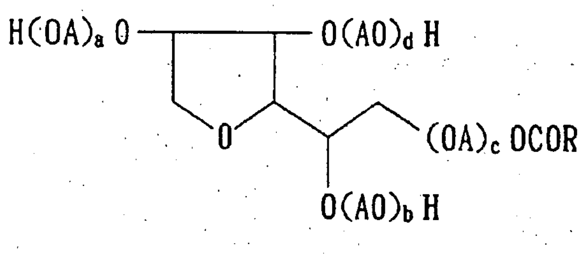

[wherein

R and A: each as defined above, and
a, b, c, d, e and f: each the number of $C_2$-$C_4$ alkylene oxide mole added]

In particular, the polyhydric alcohol ester represented by the general formula (4-I) to be used in the invention is preferably a reaction product prepared by reacting a natural fat or oil with glycerol and an alkylene oxide at a molar ratio of the fat or oil to glycerol to the alkylene oxide of 1 : (0.1 to 5) : (2 to 100). Such a reaction product can be prepared as follows.

Namely, the reaction product can be prepared by mixing a natural fat or oil with glycerol, adding a base catalyst to the obtained mixture and adding an alkylene oxide to the resulting mixture gradually to conduct a reaction.

The natural fat or oil to be used in this case is tallow, palm oil, palm kernel oil, coconut oil, a product of partial or complete hardening of such oil or fat, or a mixture of two or more of them. The alkylene oxide to be used in this case is ethylene oxide, propylene oxide or butylene oxide, preferably ethylene oxide, propylene oxide or a mixture of them. When an alkylene oxide mixture is used, the oxides may be reacted simultaneously or successively. The base catalyst usable in this case includes potassium hydroxide, sodium hydroxide and sodium methylate. The reaction temperature ranges from 100 to 200°C, preferably 140 to 170°C.

In the above reaction, it is preferable that a natural fat or oil, glycerin and an alkylene oxide be reacted at a molar ratio of 1 : (0.1 to 5) : (2 to 100) still preferably 1 : (1 to 5) : (10 to 100).

Further, the above reaction product can be prepared also as follows.

Namely, it can be prepared by mixing a fatty acid corresponding to that of a natural fat or oil with glycerol to conduct esterification and conducting the addition reaction of the obtained ester with an alkylene oxide. Alternatively, it can be prepared by conducting the addition reaction of glycerol with an alkylene oxide and esterifying the obtained adduct with a fatty acid corresponding to that of a natural fat or oil.

Desirable examples of the quaternary ammonium salt prepared in the invention include those represented by the following general formulae (4-II), (4-III), (4-IV) and (4-V), though the quaternary ammonium salt may be any one prepared from a corresponding tertiary amine:

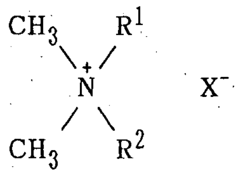

(4-II)

(wherein $R^1$ and $R^2$ represent the same or different from each other a linear or branched alkyl or alkenyl group having 8 to 36 carbon atoms; and X represents halogen or $CH_3SO_4$),

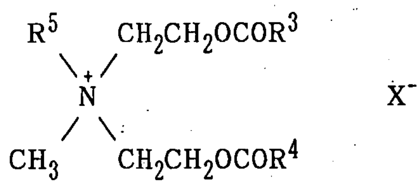

(4-III)

[wherein $R^3$ and $R^4$ represent the same or different from each other a linear or branched alkyl group having 7 to 35 carbon atoms; $R^5$ represents an alkyl or hydroxyalkyl group having 1 to 6 carbon atoms or a $-CH_2CH_2OCOR^6$ (wherein $R^6$ represents a linear or branched alkyl or alkenyl group having 7 to 35 carbon atoms); and X represents halogen or $CH_3SO_4$],

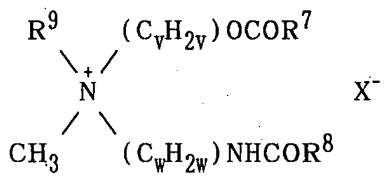

(4-IV)

(wherein $R^7$ and $R^8$ represent the same or different from each other a linear or branched alkyl or alkenyl group having 7 to 35 carbon atoms; $R^9$ represents an alkyl or hydroxyalkyl group having 1 to 6 carbon atoms; v and w are the same or different from each other an integer of 2 to 6; and X represents halogen or $CH_3SO_4$), and

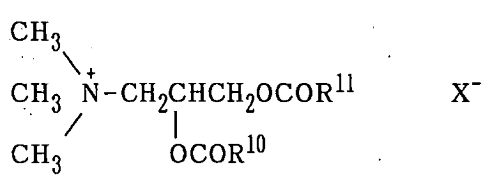

(4-V)

(wherein $R^{10}$ and $R^{11}$ represent the same or different from each other a linear or branched alkyl or alkenyl group having 7 to 35 carbon atoms; and X represents halogen or $CH_3SO_4$).

The compounds represented by the general formula (4-III), (4-IV) and (4-V) are more desirable, with those represented by the general formula (4-IV) being most desirable, which is because the softness enhancing effect of the polyhydric alcohol ester represented by the general formula (4-I) is remarkable. The reason for this phenomenon is

presumed to be that the interaction between the ester linkage of the polyhydric alcohol ester represented by the general formula (4-I) and the ester or amide linkage of the quaternary ammonium salt represented by the general formula (4-III), (4-IV) or (4-V) enhances the softness enhancing effect of the polyhydric alcohol ester represented by the general formula (4-I).

The tertiary amine to be used as the starting material in the invention includes those represented by the following general formula (4-II') to (4-V'), which are the starting materials for preparing the quaternary ammonium salts represented by the above general formula (4-II) to (4-V):

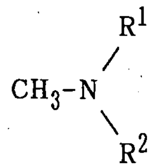
(4-II')

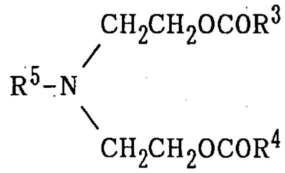
(4-III')

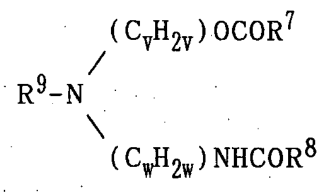
(4-IV')

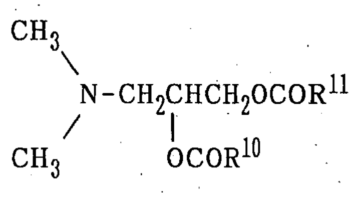
(4-V')

(wherein $R^1$, $R^2$, $R^3$, $R^4$, $R^5$, $R^7$, $R^8$, $R^9$, $R^{10}$, $R^{11}$, v and w are each as defined above).

The quaternization of a tertiary amine is conducted by mixing a corresponding tertiary amine with a polyhydric alcohol ester represented by the general formula (4-I) and adding a quaternizing agent to the obtained mixture. The weight ratio of the polyhydric alcohol ester represented by the general formula (4-I) to the tertiary amine ranges preferably from 1/99 to 99/1, still preferably 50/50 to 10/90. If necessary, an alcoholic solvent (such as isopropyl alcohol or ethanol) may be added in an amount of 0 to 20% based on the whole reactants, though the addition thereof in an amount exceeding 20% is undesirable for the object of the invention. The quaternizing agent includes chloromethane, bromomethane, iodomethane and dimethylsulfuric acid, among which chloromethane and dimethylsulfuric acid are preferable. The reaction temperature is 60 to 140°C, preferably 80 to 120°C.

The softener composition of the present invention can impart excellent softness and water absorption properties to cloth without making the cloth greasy to the touch. Further, the softener composition is excellent in biodegradability to do little harm to natural environment, and is extremely excellent in storage stability (to suffer from neither geletion nor thickening even after storage).

Example

The inventions (1), (2), (3) and (4) will now be described in this order by referring to the following

Examples,

In the Examples, all percentages are by weight unless otherwise noted, and the balance is composed of water.

Example (1)

Examples 1 to 41

Liquid softener compositions having formulations specified in Tables 9 to 12 were prepared by the use of softener base materials (1) listed in Tables 1 and 2, components (A) of the softener base material (2) listed in Table 3, components (B-1) of the softener base material (2) listed in Table 4, components (B-2) of the softener base material (2) listed in Table 5, components (C) listed in Table 6, components (D) listed in Table 7 and other components listed in Table 8.

Comparative Example 1

A liquid softener composition having a formulation specified in Table 13 was prepared by the use of a mixture comprising the softener base material (1-6) listed in Table 1 and the component (E-5) listed in Table 8 (corresponding to the mixture of Example 12 of Japanese Patent Laid-Open No. 57632/1994).

Comparative Example 2

A liquid softener composition having a formulation specified in Table 13 was prepared by the use of a mixture comprising the softener base material (B-2-3) listed in Table 5 and the component (E-6) listed in Table 8 (corresponding to the mixture of Example 4 of Japanese Patent Laid-Open No. 10263/1994).

Comparative Examples 3 to 16

Liquid softener compositions having formulations specified in Tables 13 and 14 were prepared by the use of components (A) of the softener base material (2) listed in Table 3, components (B-1) of the softener base material (2) listed in Table 4 and other components listed in Table 8.

The pieces of cloth treated with the liquid softener compositions prepared in Examples 1 to 41 and Comparative Examples 1 to 16 were evaluated for softness and greasiness by the following method. Further, the compositions were also evaluated for storage stablility by the following method. The results are given in Tables 9 to 14.

<Evaluation of softness and greasiness>

Commercially available cotton towel, acrylic fabric and polyester fabric were washed with "Attack" (a product of Kao Corporation, registered trademark) five times and thereafter freed from the detergent. The resulting towel and fabrics were treated with 0.5% (by weight based on the towel or fabric) of each of the liquid softener compositions prepared above at 25°C and a bath ratio of 1/10 under stirring for 3 minutes. The towel and fabrics thus treated were air-dried in room and allowed to stand in a thermo-hygrostatic chamber of 20°C and 65% RH for 24 hours.

The resulting towel and fabrics were evaluated for softness and gresiness.

The evaluation of softness and greasiness was conducted according to the following criteria as compared with the towel and fabrics treated with the softener composition of Comparative Example 16 (as control)

softness:

+2: softer than the control
+1: somewhat softer than the control
0: equivalent to the control
-1: the control is somewhat softer
- 2: the control is softer

greasiness:

+2: the control is greasier
+1: the control is somewhat greasier
0: equivalent to the control

- 1: somewhat greasier than the control
- 2: greasier than the control

<Evaluation of storage stability>

The liquid softener compositions prepared above were stored in a hermetically stored state at 20°C for 20 days and evaluated for appearance and fluidity with the naked eye under hermetically sealed conditions. A case wherein no change in the appearance or the fluidity was shown by "good", while a case wherein a change was observed was shown by the change.

<Evaluation of water absorption properties [Byreck method]>

The pieces of cloth treated in the same manner as that described in the section "Evaluation of softness and greasiness" were allowed to stand in a thermo-hygrostatic chamber of 25°C and 65% RH for 24 hours. The resulting cotton towel was evaluated for water absorption properties.

A rectangular piece (25 cm × 2 cm) was cut off from the non-pile area of the cotton towel. Water of 25°C was placed in the above thermo-hygrostatic chamber and this piece was dipped in the water in a state kept vertically up to 2 cm from the lower end to absorb water. After 15 minutes, the height of ascent of water was measured.

Table 1

| | Softener base material (1) | | |
|---|---|---|---|
| | Synth. process | (I)/(II)/(III)/(IV) wt. ratio | Griffin's HLB |
| 1-1 | compound prepared from methyl ester of hardened palm stearic acid, glycerol and E0* at molar ratio of 2 : 1 : 4 by process (ii) | 0.150/0.444/0.393/0.013<br>0.150/0.444/0.393/0.013 | 6.69 |
| 1-2 | compound prepared from semihardened tallow*2, glycerol and E0 at a molar ratio of 1 : 0.2 : 9.6 by process (iv) | 0.045/0.306/0.647/0. 002<br>0.045/0.306/0.647/0.002 | 7.80 |
| 1-3 | compound prepared from tallow, glycerol and E0 at a molar ratio of 1 : 1 : 8 by process (ii) | 0.301/0.449/0.199/0.051 | 7.82 |
| 1-4 | compound prepared from palm stearin, glycerol and E0 at a molar ratio of 1 : 0.5 : 12 by process (ii) | 0,164/0.444/0.375/0.017 | 9.09 |
| 1-5 | compound prepared from tallow, glycerol and E0 at a molar ratio of 2.5 : 1 : 12 by process (iii) | 0.048/0.312/0.638/0.002 | 9.43 |
| 1-6 | compound prepared from hardened tallow, glycerol and E0 at a molar ratio of 1 : 0.25 : 15 by process (ii) [the component (b-1) of Example of Japanese Patent Laid-Open No. 57632/1994] | 068/0. 352/0. 576/0. 004 | 9.63 |
| 1-7 | compound prepared from methyl ester of palm kernel oil fatty acid, glycerol and E0 at a molar ratio of 1.7 : 1 : 6 by process (iv) | 0. 255/0. 454/0. 250/0. 041 | 9.76 |

Table 2

| | Softener base material (1) | | |
|---|---|---|---|
| | Synth. process | (I)/(II)/(III)/(IV) wt. ratio | Griffin's HLB |
| 1-8 | compound prepared from tallow fatty acid, glycerol and E0 at a molar ratio of 2 : 1 : 10 by process (i) | 0.167/0.444/0.370/0.019 | 9.78 |
| 1-9 | compound prepared from methyl ester*3 of semihardened palm stearic acid, glycerol and E0 at a molar ratio of 1.8 : 1 : 10 by process (iv) | 0.230/0.454/0.282/0.035 | 10.52 |
| 1-10 | compound prepared from semihardened tallow, glycerol and E0 at a molar ratio of 1 : 0.5 : 18 by process (ii) | 0.172/0.444/0.364/0.020 | 10.54 |
| 1-11 | compound prepared from methyl ester of palm kernel oil fatty acid, glycerol and E0 at a molar ratio of 2 : 1 : 10 by process (ii) | 0.174/0.444/0.360/0.021 | 10.95 |
| 1-12 | compound prepared from hardened palm stearin, glycerol and E0 at a molar ratio of 1 : 0.25 : 20 by process (ii) | 0.072/0.357/0.567/0.005 | 11.09 |
| 1-13 | compound prepared from coconut oil fatty acid, glycerol and E0 at a molar ratio of 2 : 1 : 10 by process (iii) | 0.176/0.444/0.358/0.022 | 11.18 |
| 1-14 | compound prepared from palm oil fatty acid, glycerol and E0 at a molar ratio of 2 : 1 : 16 by process (iii) | 0. 178/0. 444/0. 355/0. 022 | 11.79 |
| 1-15 | compound prepared from palm oil, glycerol and E0 at a molar ratio of 1 : 1 : 24 by process (ii) | 0.327/0.423/0.173/0.077 | 11.97 |

notes)
*1: EO represents ethylene oxide
*2: semihardened tallow is a mixture comprising tallow and hardened tallow at a weight ratio of 1 : 1
*3: methyl ester of semihardened palm stearic acid is

a mixture comprising methyl ester of palm stearic acid and methyl ester of hardened palm stearic acid at a weight ratio of 1 : 1

Table 3

| | Component (A) of the softener base material (2) | | |
|---|---|---|---|
| | Synth. process | (V)/(VI)/(VII)/(VIII) wt. ratio | Griffin's HLB |
| A-1 | compound prepared from methyl ester of tallow fatty acid, glycerol and E0 at a molar ratio of 0.6 : 1 : 3 by process (iv) | 0.489/0.188/0.021/0.302 | 11.46 |
| A-2 | compound prepared from palm stearin, glycerol and E0 at a molar ratio of 1 : 4.45 : 16.36 by process (i) | 0.480/0.165/0.017/0.338 | 12.09 |
| A-3 | compound prepared from hardened tallow fatty acid, glycerol and E0 at a molar ratio of 2 : 1 : 20 by process (i) | 0.183/0.444/0.348/0.024 | 12.72 |
| A-4 | compound prepared from hardened palm stearin, glycerol and E0 at a molar ratio of 1 : 0.36 : 40.91 by process (iii) | 0.133/0.419/0.434/0.014 | 14. 14 |
| A-5 | compound prepared from ethyl laurate, glycerol and E0 at a molar ratio of 0.55 : 1 : 4 by process (ii) | 0.448/0.141/0.014/0.397 | 14.18 |
| A-6 | compound prepared from coconut oil, glycerol and E0 at a molar ratio of 1 : 2 : 36 by process (i) | 0.444/0.275/0.055/0.226 | 14.94 |

Table 4

| Component (B-1) of the softener base material (2) | |
|---|---|
| B-1-1*1 | 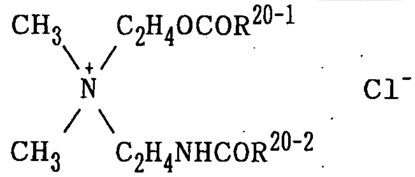 |
| B-1-2*2 | 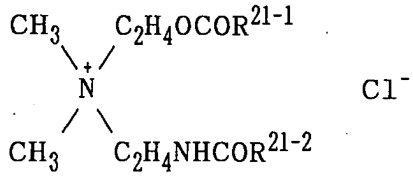 |
| B-1-3 | 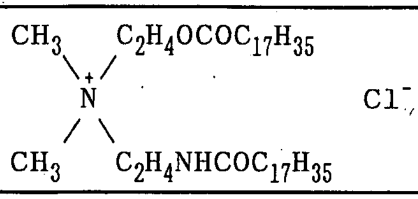 |
| B-1-4 | 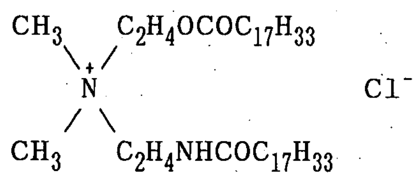 |

notes)

*1: $R^{20-1}$ and $R^{20-2}$ each represent an alkyl group resulting from hardened tallow fatty acid

*2: $R^{21-1}$ and $R^{21-2}$ each represent an alkyl group resulting from hardened palm oil fatty acid

Table 5

| Component (B-2) of the softener base material (2) | |
|---|---|
| B-2-1[*1] | 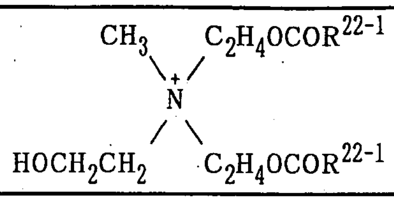 $Cl^-$ |
| B-2-2[*1] | mixture of the following components (weight ratio of monoester to diester to triester: 20 : 55 : 25)<br>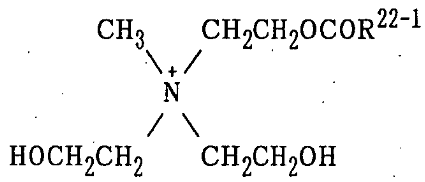 $CH_3SO_4^-$<br>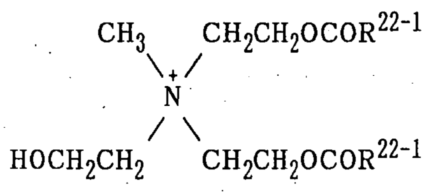 $CH_3SO_4^-$<br>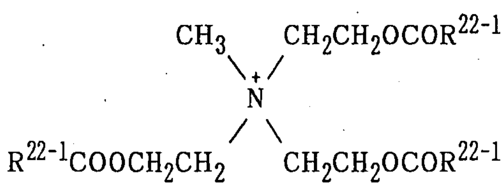 $CH_3SO_4^-$ |

Table 5 (cont'd)

| Component (B-2) of the softener base material (2) | |
|---|---|
| B-2-3*2 | mixture of the following components (weight ratio of monoester to diester to triester: 20 : 55 : 25)<br>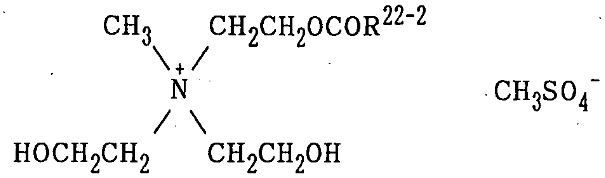<br>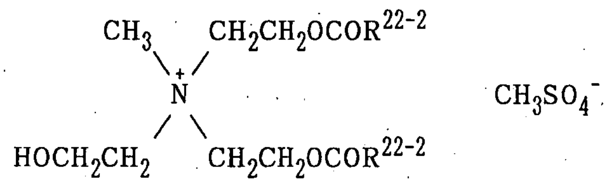<br>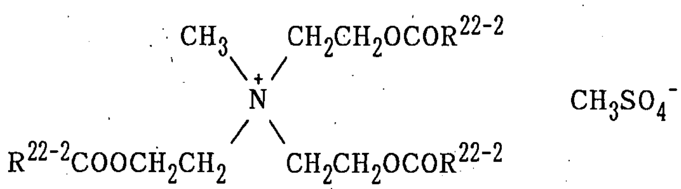 |

notes)

*1: $R^{22-1}$ represents an alkyl group resulting from hardened tallow fatty acid

*2: $R^{22-2}$ represents an alkyl group resulting from a mixture comprising unhardened tallow fatty acid and hardened tallow fatty acid at a weight ratio of 75 : 25

Table 6

| Component (C) | |
|---|---|
| C-1 | adduct of glycerol with E0 (MW 8,900) |
| C-2 | adduct of glycerol with PO/EO (15 : 85) (MW 10,000) |
| C-3 | adduct of sorbitol with PO/EO (10 : 90) (MW 15,000) |
| C-4 | adduct of tetraethylenepentamine with PO/EO (2 : 98) (MW 20,000) |
| C-5 | adduct of polyethyleneimine (MW: 3000) with PO/EO (5 : 95) (MW 300,000) |

Table 7

| Component (D) | |
|---|---|
| D- 1 | hardened tallow fatty acid |
| D- 2 | semihardened tallow fatty acid (wt. ratio of tallow to hardened tallow: 75 : 25) |
| D- 3 | palm oil fatty acid |
| D- 4 | palm stearic acid |
| D- 5 | stearic acid |
| D- 6 | oleic acid |
| D- 7 | hardened palm oil fatty acid |
| D- 8 | palm kernel oil fatty acid |
| D- 9 | coconut oil fatty acid |
| D-10 | lauric acid |
| D-11 | tallow fatty acid |
| D-12 | hardened palm stearic acid |

Table 8

| Other components | |
|---|---|
| E-1 | ethanol |
| E-2 | isopropanol |
| E-3 | polyoxyethylene (20 mole) layryl ether |
| E-4 | polyoxyethylene(20 mole)stearylamine |
| E-5 | 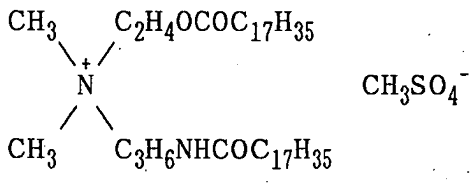<br>[the component (a-3) of Example of Japanese Patent Laid-Open No. 57632/1994] |
| E-6 | product of the reaction of lard oil with 15 wt% of propylene oxide<br>[the component described in Example 4 of Japanese Patent Laid-Open No. 10263/1994] |
| E-7*1 | compound prepared by reacting tallow fatty acid with glycerol and EO at a molar ratio of 1.8 : 1 : 50 by process (i)<br>(I)/(II)/(III)/(IV) w ratio :<br>0.266/0.440/0.240/0.053<br>HLB=16.42 |
| E-8 | compound prepared by reacting hardened palm oil with glycerol and EO at a molar ratio of 1 : 0.111 : 20 by process (ii)<br>(I)/(II)/(III)/(IV) wt. ratio:<br>0.019/0.215/0.765/0.001<br>HLB=10.83 |
| E-9 | compound prepared by reacting palm kernel oil with glycerol and EO at a molar ratio of 1 : 0.111 : 6.67 by process (ii)<br>(I)/(II)/(III)/(IV) wt. ratio:<br>0.017/0.205/0.778/0.000<br>HLB=7.5 |

note)

*1: EO represents ethylene oxide

Table 9

| | | Softener base material (1) | Softener base material (2) | | Component (C) | Component (D) | Other component | Softness | Greasiness | Storage stabilty | Water absorption properties (cm) |
|---|---|---|---|---|---|---|---|---|---|---|---|
| | | | Component (A) | Component (B) | | | | | | | |
| Ex. | 1 | 1-9 (20) | - | - | - | - | - | 0 | +2 | good | 15.5 |
| | 2 | 1-10 (20) | - | - | - | - | - | 0 | +2 | good | 15.5 |
| | 3 | 1-1 (20) | - | - | - | - | E-3 (3) | 0 | +2 | good | 13.2 |
| | 4 | 1-2 (20) | - | - | - | - | E-3 (3) | 0 | +2 | good | 14.0 |
| | 5 | 1-3 (20) | - | - | - | - | E-3 (3) | 0 | +2 | good | 14.5 |
| | 6 | 1-4 (20) | - | - | - | - | E-3 (2) | 0 | +2 | good | 14.5 |
| | 7 | 1-5 (20) | - | - | - | - | E-3 (2) | 0 | +2 | good | 15.0 |
| | 8 | 1-6 (20) | - | - | - | - | E-3 (2) | 0 | +2 | good | 14.5 |
| | 9 | 1-7 (20) | - | - | - | - | E-3 (2) | 0 | +2 | good | 15.2 |
| | 10 | 1-8 (20) | - | - | - | - | E-3 (2) | 0 | +2 | good | 15.2 |
| | 11 | 1-9 (18) | - | - | - | D-3 (2) | E-3 (2) | 0 | +2 | good | 15.0 |

Table 10

| | | Softener base material (1) | Softener base material (2) | | Component (C) | Component (D) | Other component | Softness | Greasiness | Storage stabilty | Water absorption properties (cm) |
|---|---|---|---|---|---|---|---|---|---|---|---|
| | | | Component (A) | Component (B) | | | | | | | |
| Ex. | 12 | 1-10 (18) | - | - | - | D-9 (2) | E-3 (2) | 0 | +2 | good | 15.0 |
| | 13 | 1-11 (19.5) | - | - | - | D-8 (0.5) | - | 0 | +2 | good | 15.7 |
| | 14 | 1-12 (19.5) | - | - | - | D-12 (0.5) | - | 0 | +2 | good | 15.2 |
| | 15 | 1-13 (19.5) | - | - | - | D-10 (0.5) | - | 0 | +2 | good | 15.9 |
| | 16 | 1-14 (19) | - | - | - | D-1 (1) | - | 0 | +2 | good | 15.9 |
| | 17 | 1-15 (18.5) | - | - | - | D-4 (1.5) | - | 0 | +2 | good | 16.0 |
| | 18 | - | A-1 (4) | B-1-1 (16) | - | - | E-2 (2) | +2 | +2 | good | 13.0 |
| | 19 | - | A-2 (4) | B-1-1 (16) | - | - | E-2 (2) | +2 | +2 | good | 13.5 |
| | 20 | - | A-3 (4) | B-1-1 (16) | - | - | E-2 (2) | +2 | +2 | good | 13.2 |
| | 21 | - | A-4 (4) | B-1-1 (16) | - | - | E-2 (2) | +2 | +2 | good | 13.2 |
| | 22 | - | A-5 (4) | B-1-1 (16) | - | - | E-2 (2) | +2 | +2 | good | 13.2 |

Table 11

| | | Softener base material (1) | Softener base material (2) | | Component (C) | Component (D) | Other component | Softness | Greasiness | Storage stabilty | Water absorption properties (cm) |
|---|---|---|---|---|---|---|---|---|---|---|---|
| | | | Component (A) | Component (B) | | | | | | | |
| Ex. | 23 | - | A-6 (4) | B-1-1 (16) | - | - | E-2 (2) | +2 | +2 | good | 13.7 |
| | 24 | 1-15 (4) | - | B-1-1 (16) | - | - | E-2 (2) | +2 | +2 | good | 13.5 |
| | 25 | 1-15 (4) | - | B-1-2 (16) | - | - | E-2 (2) | +2 | +2 | good | 13.5 |
| | 26 | 1-15 (4) | - | B-1-3 (16) | - | - | E-2 (2) | +2 | +2 | good | 13.6 |
| | 27 | 1-15 (4) | - | B-1-4 (16) | - | - | E-2 (2) | +2 | +2 | good | 14.5 |
| | 28 | - | A-1 (4) | B-2-3 (16) | - | - | E-2 (2) | +2 | +2 | good | 13.2 |
| | 29 | - | A-2 (4) | B-2-3 (16) | - | - | E-2 (2) | +2 | +2 | good | 13.7 |
| | 30 | - | A-3 (4) | B-2-3 (16) | - | - | E-2 (2) | +2 | +2 | good | 13.5 |
| | 31 | - | A-4 (4) | B-2-3 (16) | - | - | E-2 (2) | +2 | +2 | good | 13.5 |
| | 32 | - | A-5 (4) | B-2-3 (16) | - | - | E-2 (2) | +2 | +2 | good | 13.5 |
| | 33 | - | A-6 (4) | B-2-3 (16) | - | - | E-2 (2) | +2 | +2 | good | 14.0 |

Table 12

| | | Softener base material (1) | Softener base material (2) | | Component (C) | Component (D) | Other component | Softness | Greasiness | Storage stabilty | Water absorption properties (cm) |
|---|---|---|---|---|---|---|---|---|---|---|---|
| | | | Component (A) | Component (B) | | | | | | | |
| Ex. | 34 | 1-15 (4) | - | B-2-1 (16) | - | - | E-2 (2) | +2 | +2 | good | 13.2 |
| | 35 | 1-15 (4) | - | B-2-2 (16) | - | - | E-2 (2) | +2 | +2 | good | 13.2 |
| | 36 | 1-15 (4) | - | B-2-3 (16) | - | - | E-2 (2) | +2 | +2 | good | 13.5 |
| | 37 | 1-5 (2) | - | B-1-1 (18) | C-1 (1.5) | D-2 (4) | E-2 (2) E-3 (2) | +2 | +2 | good | 13.2 |
| | 38 | 1-5 (2) | - | B-1-1 (18) | C-2 (1.5) | D-5 (4) | E-2 (2) E-3 (2) | +2 | +2 | good | 13.0 |
| | 39 | 1-5 (2) | - | B-1-1 (18) | C-3 (1.5) | D-6 (4) | E-2 (2) E-3 (2) | +2 | +2 | good | 13.2 |
| | 40 | 1-10 (3) | - | B-2-3 (17) | C-4 (1.5) | D-7 (4) | E-2 (2) E-3 (1) E-4 (1) | +2 | +2 | good | 13.0 |
| | 41 | 1-10 (3) | - | B-2-3 (17) | C-5 (1.5) | D-11 (4) | E-2 (2) E-3 (1) E-4 (1) | +2 | +2 | good | 13.2 |

Table 13

| | | Softener base material (1) | Softener base material (2) | | Component (C) | Component (D) | Other component | Softness | Greasiness | Storage stabilty | Water absorption properties (cm) |
|---|---|---|---|---|---|---|---|---|---|---|---|
| | | | Component (A) | Component (B) | | | | | | | |
| Comp. Ex. | 1 | - | A-1 (2) | - | - | - | E-5 (18) | -1 | -1 | good | 11.0 |
| | 2 | - | - | B-2-3 (16) | - | - | E-6 (4) | -1 | +1 | separati-on | 11.5 |
| | 3 | - | A-1 (20) | - | - | - | - | -1 | +2 | separati-on | 15.9 |
| | 4 | - | A-2 (20) | - | - | - | - | -1 | +2 | separati-on | 16.0 |
| | 5 | - | A-3 (20) | - | - | - | - | -2 | +2 | separati-on | 16.0 |
| | 6 | - | A-4 (20) | - | - | - | - | -2 | +2 | separati-on | 16.0 |
| | 7 | - | A-5 (20) | - | - | - | - | -2 | +2 | separati-on | 16.0 |
| | 8 | - | A-6 (20) | - | - | - | - | -2 | +2 | separati-on | 16.0 |
| | 9 | - | - | B-1-1 (16) | - | - | E-1 (2) E-3 (2) E-7 (4) | -1 | +2 | good | 13.7 |
| | 10 | - | - | B-2-3 (16) | - | - | E-1 (2) E-3 (2) E-7 (4) | -1 | +2 | good | 14.0 |

Table 14

| | | Softener base material (1) | Softener base material (2) | | Component (C) | Component (D) | Other component | Softness | Greasiness | Storage stabilty | Water absorption properties (cm) |
|---|---|---|---|---|---|---|---|---|---|---|---|
| | | | Component (A) | Component (B) | | | | | | | |
| Comp. Ex. | 11 | - | - | B-1-1 (16) | - | - | E-2 (2)<br>E-3 (2)<br>E-8 (4) | 0 | +2 | separation | 12.0 |
| | 12 | - | - | B-2-3 (16) | - | - | E-2 (2)<br>E-3 (2)<br>E-8 (4) | 0 | +2 | separation | 13.2 |
| | 13 | - | - | B-1-1 (16) | - | - | E-2 (2)<br>E-3 (2)<br>E-9 (4) | 0 | +2 | separation | 11.0 |
| | 14 | - | - | B-2-3 (16) | - | - | E-2 (2)<br>E-3 (2)<br>E-9 (4) | 0 | +2 | separation | 11.2 |
| | 15 | - | - | B-1-1 (20) | - | - | E-2 (2) | +2 | -2 | good | 10.5 |
| | 16 | - | - | B-2-3 (20) | - | - | E-2 (2) | 0 | 0 | gelation | 10.8 |

note) In Tables 9 to 14, the figures in parentheses each represent the content of a corresponding component in the composition and the unit thereof is % by weight. The balance is composed of water.

Example (2)

Examples 2-1 to 2-11

Liquid softener compositions having formulations specified in Table 2-6 were prepared by the use of components (2-A) listed in Table 2-1, components (2-B) listed in Table 2-2, components (2-C) listed in Table 2-3, components (2-D) listed in Table 2-4 and the other components listed in Table 2-5.

Comparative Examples 2-1 to 2-4

Liquid softener compositions having formulations specified in Table 2-6 were prepared by the use of component (2-A) listed in Table 2-1, component (2-B) listed in Table 2-2 and the other components listed in Table 2-5.

The pieces of cloth treated with the liquid softener compositions prepared in Examples 2-1 to 2-11 and Comparative Examples 2-1 to 2-4 were evaluated for softness and greasiness in the same manner as that of Example (1). Further, the compositions were evaluated also for storage stability by the following method. The results are given in Table 2-6.

The evaluation of storage stability was conducted by storing each liquid softener composition in a hermetically sealed state at 20°C and 40°C for 20 days.

Table 2-1

| | Component (2-A) | |
|---|---|---|
| | $R^1$ in the formula (2-I) | No. of alkylene oxide mole added to diethanolamide |
| 2-A-1 | alkyl group resulting from hardened tallow fatty acid | 0 |
| 2-A-2 | alkyl group resulting from semihardened tallow fatty acid (wt. ratio of hardened tallow to tallow: 1/1) | 0 |
| 2-A-3 | alkyl group resulting from palm stearic acid | 0 |
| 2-A-4 | alkyl group resulting from hardened palm stearic acid | ethylene oxide 2 |
| 2-A-5 | alkyl group resulting from coconut oil fatty acid | 0 |
| 2-A-6 | alkyl group resulting from palm kernel oil fatty acid | 0 |
| 2-A-7 | alkyl group resulting from lauric acid | propylene oxide 1 |

Table 2-2

| Component (2-B) | |
|---|---|
| 2-B-1*1 | 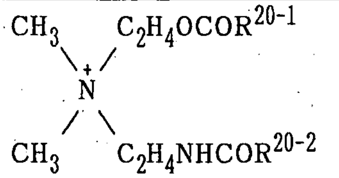 $Cl^-$ |
| 2-B-2*2 | 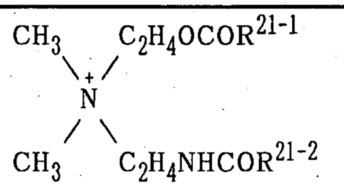 $Cl^-$ |
| 2-B-3 | 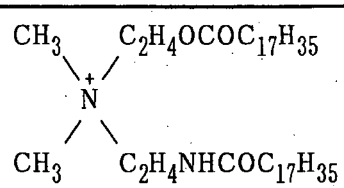 $Cl^-$ |
| 2-B-4 | 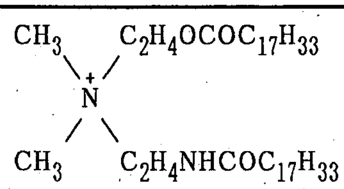 $Cl^-$ |

notes)

*1: $R^{20-1}$ and $R^{20-2}$ each represent an alkyl group resulting from hardened tallow fatty acid

*2: $R^{21-1}$ and $R^{21-2}$ each represent an alkyl group resulting from hardened palm oil fatty acid

Table 2-3

| Component (2-C) | |
|---|---|
| 2-C-1 | adduct of glycerol with EO (MW 8,900) |
| 2-C-2 | adduct of glycerol with PO/EO (15 : 85) (MW 10,000) |
| 2-C-3 | adduct of sorbitol with PO/EO (10 : 90) (MW 15,000) |
| 2-C-4 | adduct of tetraethylenepentamine with PO/EO (2 : 98) (MW 20,000) |
| 2-C-5 | adduct of polyethyleneimine (MW: 3,000) with PO/EO (5 : 95) (MW 300,000) |

Table 2-4

| | Component (2-D) |
|---|---|
| 2-D-1 | tallow fatty acid |
| 2-D-2 | palm stearic acid |
| 2-D-3 | oleic acid |

Table 2-5

| Other components | |
|---|---|
| 2-E-1 | isopropanol |
| 2-E-2 | polyoxyethylene (20 mole) lauryl ether |
| 2-E-3*1 | 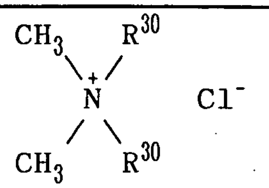 |
| 2-E-4*2 | adduct of hardened tallow fatty acid diethanolamide with 8 EO molecules |
| 2-E-5 | tallow fatty acid monoethanolamide |
| 2-E-6*3 | quaternization product of partially hardened tallow fatty acid ester of triethanolamine |

notes)

*1: $R^{30}$ represents an alkyl group resulting from hardened tallow fatty acid

*2: EO represents ethylene oxide

*3: mixture comprising 20 wt% of a compound represented by the following formula (2-E-6-1), 45 wt% of a compound represented by the formula (2-E-6-2) and 35 wt% of a compound represented by the formula (2-E-6-3):

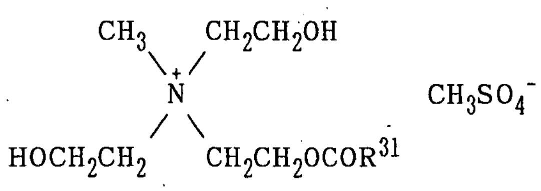

(2-E-6-1)

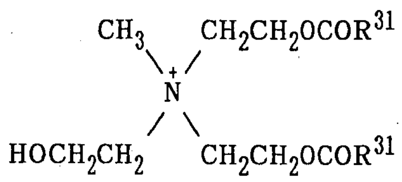 $CH_3SO_4^-$ (2-E-6-2)

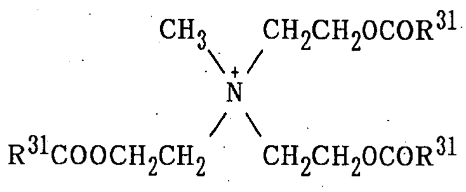 $CH_3SO_4^-$ (2-E-6-3)

(wherein $R^{31}$ represents a mixed group comprising alkyl resulting from hardened tallow fatty acid and alkyl resulting from unhardened tallow fatty acid at a wt. ratio of 25 : 75)

Table 2-6

| | | Component (2-A) | Component (2-B) | Component (2-C) | Component (2-D) | Other component | Softness | Greasiness | Storage stabilty | |
|---|---|---|---|---|---|---|---|---|---|---|
| | | | | | | | | | 40°C | 20°C |
| Ex. | 2-1 | 2-A-1 (4) | 2-B-1 (16) | 2-C-1 (0.5) | - | 2-E-1 (2)<br>2-E-2 (2) | +2 | +2 | good | good |
| | 2-2 | 2-A-2 (4) | 2-B-1 (16) | - | - | 2-E-1 (2) | +2 | +2 | good | good |
| | 2-3 | 2-A-3 (4) | 2-B-1 (16) | 2-C-2 (0.5) | - | 2-E-1 (2) | +2 | +2 | good | good |
| | 2-4 | 2-A-4 (4) | 2-B-1 (16) | - | - | 2-E-1 (2) | +1 | +2 | good | good |
| | 2-5 | 2-A-5 (4) | 2-B-1 (16) | - | - | 2-E-1 (2)<br>2-E-2 (2) | +2 | +2 | good | good |
| | 2-6 | 2-A-6 (4) | 2-B-1 (16) | - | - | 2-E-1 (2)<br>2-E-2 (2) | +2 | +2 | good | good |
| | 2-7 | 2-A-7 (4) | 2-B-1 (16) | 2-C-3 (0.5) | - | 2-E-1 (2)<br>2-E-2 (2) | +2 | +2 | good | good |
| | 2-8 | 2-A-2 (2) | 2-B-2 (16) | 2-C-4 (0.5) | 2-D-1 (1.5) | 2-E-1 (2) | +2 | +2 | good | good |
| | 2-9 | 2-A-6 (2) | 2-B-3 (16) | - | 2-D-2 (1.5) | 2-E-1 (2) | +2 | +2 | good | good |
| | 2-10 | 2-A-7 (2) | 2-B-4 (16) | - | 2-D-3 (1.5) | 2-E-1 (2) | +2 | +2 | good | good |
| | 2-11 | 2-A-2 (4) | 2-B-2 (16) | 2-C-5 (0.5) | - | 2-E-1 (2) | +2 | +2 | good | good |
| Comp. Ex. | 2-1 | 2-A-2 (4) | - | - | - | 2-E-6 (16) | -2 | +2 | gelation | good |
| | 2-2 | - | - | - | - | 2-E-3 (20)<br>2-E-1 (3) | 0 | 0 | gelation | good |
| | 2-3 | - | 2-B-1 (16) | - | - | 2-E-4 (4) | -1 | +1 | good | good |
| | 2-4 | - | 2-B-1 (16) | - | - | 2-E-5 (4) | 0 | 0 | separation | separation |

note)

In Table 2-6, the figures in parentheses each represent the content of a corresponding component in the composition and the unit thereof is % by weight. The balance is composed of water.

The invention (3) will now be described in more detail by referring to the following Examples.

Synthesis Example 3-1

Diethanoldmine (172 g) and 5 g of a 28% methanolic solution of $NaOCH_3$ were put in a four-necked flask fitted with a stirrer and a thermometer. The contents were stirred at 100°C under a pressure of 30 Torr for one hour to distill away the methanol. 417 g of purified palm stearin oil was added to the residue and the obtained mixture was reacted at 100°C under a pressure of 30 Torr for 2 hours, cooled to 60°C and thereafter aged for 50 hours to give diethanolamide resulting from palm stearin oil.

Synthesis Example 3-2

180 of the diethanolamide resulting from palm stearin oil prepared in Synthesis Example 3-1 was charged into an autoclave and heated to 150°C, followed by the addition of 66 g of ethylene oxide in 2 hours. The resulting mixture was aged for one hour to give an adduct of diethanolamide resulting from palm stearin oil with 3 ethylene oxide moles.

Synthesis Examples 3-3 to 3-6

Diethanolamides were prepared by the use of the starting materials listed in Table 3-1 under the conditions specified in Table 3-1 with the other conditions being the same as those of Synthesis Example 3-1.

Table 3-1

| Synth. Ex. No. | 3-1 | 3-2 | 3-3 | 3-4 | 3-5 | 3-6 |
|---|---|---|---|---|---|---|
| Starting material | palm stearin oil 417 g | palm stearin oil 417 g | hardened palm stearin oil 418 g | hardened tallow 602 g | palm kernel oil 674 g | methyl laurate 642 g |
| diethanolamine | 172 g | 172 g | 156 g | 77 g | 335 g | 330 g |
| 28% methanolic solution of $NaOCH_3$ | 5 g | 5 g | 5 g | 6 g | 10 g | 10 g |
| Reaction temp. | 100°C | 100°C | 100°C | 120°C | 120°C | 110°C |
| Reaction pressure | 30 torr | 30 torr | 30 torr | 50 torr | 50 torr | 50 torr |
| Reaction time | 2 hr | 2 hr | 2 hr | 2 hr | 3 hr | 3 hr |
| Alkylene oxide | 0 | EO* 3 mol | 0 | 0 | 0 | 0 |

note)

*: EO represents ethylene oxide

Example 3-1

170 g of the reaction product prepared in Synthesis Example 3-1 and 630 g of N-methyl-N-(hardened tallow alkanoyloxyethyl)-N-(hardened tallow alkanoylaminoethyl)amine were charged into an autoclave fitted with a stirrer and a thermometer, followed by the injection of 61 g of chloromethane. The contents were heated to 90°C and reacted at that temperature for 6 hours to give a reaction mixture mainly comprising N,N-dimethyl-N-(hardened tallow alkanoyloxyethyl)-N-(hardened tallow alkanoyl-aminoethyl)ammonium chloride.

Examples 3-2 to 3-4

Reaction mixtures each containing'a quaternary ammonium salt listed in Table 3-2 as the main component were prepared by the use of tertiary amines listed in Table 3-2 under the conditions specified in Table 3-2, with the other conditions being the same as those of Example 3-1.

Comparative Examples 3-1 to 3-4

Reaction mixtures each containing a quaternary ammonium salt listed in Table 3-2 as the main component were prepared in the same manners as those of Examples 3-1 to 3-4 except that the reaction product prepared in Synthesis Example 3-1, 3-2, 3-3 or 3-4 was replaced by the same amount of isopropyl alcohol.

The reaction conditions of Examples 3-1 to 3-4 and Comparative Examples 3-1 to 3-4 and the structures of the quaternary ammonium salts prepared in the Examples and Comparative Examples are given in Table 3-2 together with the results of evaluation softness and smell as determined by the following method.

<Evaluation of softness and smell>

(1) Preparation of dispersion of quaternary ammonium salt

A 5% by weight dispersion of a quaternary ammonium salt was prepared by melting the reaction mixture prepared in the above Example or Comparative Example and containing a quaternary ammonium salt as the main component and dropping the obtained melt into water under stirring.

(2) Method of treatment

A commercially available cotton towel (2 kg) was washed with a commercially available detergent "Attack" (a product of Kao Corporation, registered trademark) and hard water of 3.5° DH by the use of a 30-1 washing machine five times, followed by the throwing of 25 ml of the above quaternary ammonium salt dispersion into the washing machine tub. The contents were stirred at 25°C for one minute to conduct treatment.

(3) Evaluation of softness

The cloth treated above was air-dried at room temperature and allowed to stand in a thermo-hygrostatic chamber of 25°C and 65 %RH for 24 hours. The resulting cloth was evaluated for softness according to the following criteria by using the cloth treated with the quaternary ammonium salt of Comparative Example 1 as control:

3: pretty softer than the control
2: softer than the control
1: somewhat softer than the control
0: equivalent to the control in softness

(4) Evaluation of smell

The 5% by weight dispersions of quaternary ammonium salts prepared above were evaluated for smell by nosing according to the following criteria:

×: alcoholic stench
o: no alcoholic stench

Table 3-2

| | Ex. 3-1 | Comp. Ex. 3-1 | Ex. 3-2 | Comp. Ex. 3-2 | Ex. 3-3 | Comp. Ex. 3-3 | Ex. 3-4 | Comp. Ex. 3-4 |
|---|---|---|---|---|---|---|---|---|
| Tert. amine | 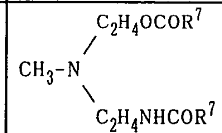 $-COR^7$; resulting from hardened tallow 630 g | | 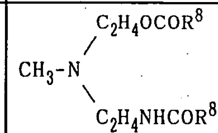 $-R^8$; $C_{17}H_{35}$ 650 g | | 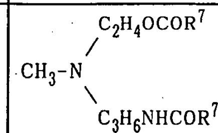 $-COR^7$; resulting from hardened tallow 644 g | | 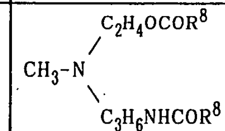 $-R^8$; $C_{17}H_{35}$ 664 g | |
| Compd. of Synth. Ex. | product of Synth. Ex. 3-1 170 g | none | product of Synth. Ex. 3-2 230 g | none | product of Synth. Ex. 3-3 170 g | none | product of Synth. Ex. 3-4 170 g | none |
| Isopropyl alcohol | none | 170 g | none | 230 g | none | 170 g | none | 170 g |
| Quaternizing agent | $CH_3Cl$ 61 g | | $CH_3Cl$ 61 g | | $CH_3Cl$ 61 g | | $CH_3Cl$ 61 g | |
| Reaction temp. | 90°C | | 90°C | | 90°C | | 90°C | |
| Reaction time | 6 hr | | 6 hr | | 6 hr | | 6 hr | |
| Quaternary ammonium salt formed | 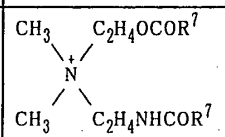 $Cl^-$ | | 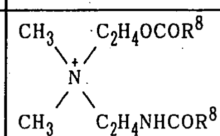 $Cl^-$ | | 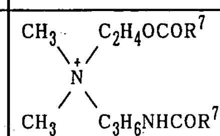 $Cl^-$ | | 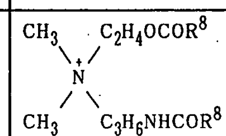 $Cl^-$ | |
| Results of evaluation of softness | 3 | 0 | 3 | 0 | 2 | 0 | 3 | 0 |
| Results of evaluation of smell | ○ | × | ○ | × | ○ | × | ○ | × |

As apparent from the results given in Table 3-2, the reaction mixtures of Example are superior to those of Comparative Examples in both softening effect and smell.

Example 3-5

85 g of the reaction product of Synthesis Example 3-5, 85 g of isopropyl alcohol and 630 g of N-methyl-N-(hardened tallow alkanoyloxyethyl)-N-(hardened tallow alkanoylaminoethyl)amine were charged into an autoclave fitted with a stirrer and a thermometer, followed by the injection of 61 g of chloromethane. The contents were heated to 90°C and reacted at that temperature for 6 hours to give a reaction mixture mainly comprising N,N-dimethyl-N-(hardened tallow alkanoyloxyethyl)-N-(hardened tallow alkanoylaminoethyl)ammonium chloride.

Example 3-6

170 g of the reaction product of Synthesis Example 3-6 and 650 g of N-methyl-N-(octadecanoyloxyethyl) -N-(octadecanoylaminoethyl)amine were charged into a four-necked flask fitted with a stirrer, a thermometer and a condenser. The contents were heated to 70°C to conduct dissolution. 120 g of dimethylsulfuric acid was dropped into the resulting solution in 2 hours and the resulting mixture was aged at that temperature for 3 hours to give a reaction mixture mainly comprising a quaternary ammonium salt listed in Table 3-3.

Example 3-7

A reaction mixture mainly comprising a quaternary ammonium salt listed in Table 3-3 was prepared by the use of a tertiary amine listed in Table 3-3 under the conditions specified in Table 3-3 with the other conditions being the same as those of Example 3-6.

The reaction conditions of Examples 3-5 to 3-7 and the structures of the obtained quaternary ammonium salts are given in Table 3-3 together with the results of evaluation of softness and smell as determined in the same manners as those of Example 3-1.

Table 3-3

| | Ex. 3-5 | Ex. 3-6 | Ex. 3-7 |
|---|---|---|---|
| Tert. amine | 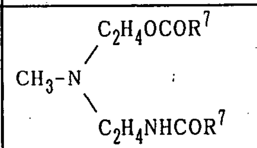<br>$-COR^7$; resulting from hardened tallow<br>630 g | 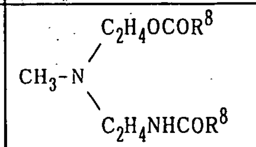<br>$-R^8$; $C_{17}H_{35}$<br>650 g | 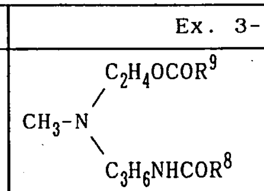<br>$-R^8$; $C_{17}H_{35}$<br>$-R^9$; $C_{11}H_{23}$<br>630 g |
| Comnpd. of Synth. Ex. | reaction product of Synth. Ex. 3-5<br>85 g | reaction product of Synth. Ex. 3-6<br>170 g | reaction product of Synth. Ex. 3-6<br>100 g |
| Alcoholic solvent | isopropyl alcohol 85 g | none | isopropyl alcohol 50 g |
| Quaternizing agent | chloromethane<br>61 g | dimethylsulfuric acid<br>120 g | dimethylsulfuric acid<br>120 g |
| Reaction temp. | 90°C | 70°C | 70°C |
| Reaction time | 6 hr | 5 hr | 6 hr |
| Quaternary ammonium salt formed | 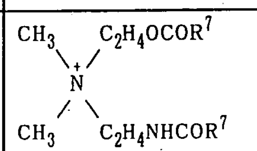 $Cl^-$ | 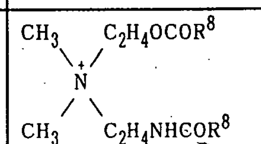 $CH_3SO_4^-$ | 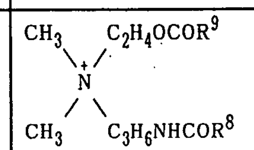 $CH_3SO_4^-$ |
| Results of evaluation of softness | 2 | 3 | 1 |
| Results of evaluation of smell | ○ | ○ | ○ |

The invention (4) will now be described in more detail by referring to the following Examples.

Synthesis Example 4-1

Tallow (85.9 g), glycerol (9.2 g) and 0.40 g of 85% potassium hydroxide were charged into an autoclave fitted with a stirrer and a thermometer. The contents were kept at 110°C under a pressure of 200 Torr for one hour to conduct dehydration. Thereafter, the system was hermetically sealed, heated to 160°C and reacted at that temperature for 3 hours. 105.6 g of ethylene oxide was added to the system in 2 hours. The obtained mixture was aged for one hour, cooled and neutralized the catalyst by the addition of 0.36 g of acetic acid to give a reaction product.

Synthesis Examples 4-2 to 4-6

Reaction products were each prepared from a natural fat or oil, glycerol and an alkylene oxide under the conditions specified in Table 4-1 with the other conditions being the same as those of Example 4-1.

Table 4-1

| | Synth. Ex. | | | | | |
|---|---|---|---|---|---|---|
| | 4-1 | 4-2 | 4-3 | 4-4 | 4-5 | 4-6 |
| Fat or oil | tallow 85.9 g | tallow 85.9 g | tallow 85.9 g | palm oil 82.9 g | palm kernel oil 67.7 g | coconut oil 65.2 g |
| Glycerol | 9.2 g | 41.9 g | 4.6 g | 1.8 g | 18.4 g | 27.6 g |
| 85% KOH | 0.4 g | 0.4 g | 0.4 g | 0.4 g | 0.4 g | 0.4 g |
| Alkylene oxide | EO 105.6 g | EO 240.2 g | EO 44.0 g PO 46.4 g | EO 10.6 g | EO 154.0 g | PO 290.0 g |
| Reaction temp. | 160°C | 160°C | 160°C | 160°C | 160°C | 160°C |

note)

alkylene oxide: EO represents ethylene oxide, and PO propylene oxide

In Synthesis Example 4-3, ethylene oxide and propylene oxide were added simultaneously.

Example 4-1

146 g of the reaction product of Synthesis Example 4-1 and 535 g of N,N-di(hardened tallow alkyl)-N-methylamine were charged into an autoclave fitted with a stirrer and a thermometer, followed by the injection of 61 g of chloromethane. The contents were heated to 90°C and reacted at that temperature for 6 hours to give N,N-di(hardened tallow alkyl)-N,N-dimethylammonium chloride. Examples 4-2 to 4-4

A quaternary ammonium salts listed in Table 4-2 were each prepared in a similar manner to that of Example 4-1 under the conditions specified in Table 4-2.

Comparative Examples 4-1 to 4-4

Quaternary ammonium salts were each prepared in the same manner as that of Example 4-1, 4-2, 4-3 or 4-4 except that isopropyl alcohol was used instead of the reaction product of Synthesis Example 4-1, 4-2 or 4-3.

The reaction conditions of Examples 4-1 to 4-4 and Comparative Examples 4-1 to 4-4 and the structures of the obtained quaternary ammonium salts are given in Table 4-2 together with the results of evaluation of softness and smell.

The evaluation of softness and smell were conducted in the same manners as those of Example 3-1.

Table 4-2

| | Ex. 4-1 | Comp. Ex. 4-1 | Ex. 4-2 | Comp. Ex. 4-2 | Ex. 4-3 | Comp. Ex. 4-3 | Ex. 4-4 | Comp. Ex. 4-4 |
|---|---|---|---|---|---|---|---|---|
| Tert. amine compound | 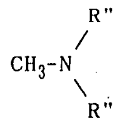 -R"; hardened tallow alkyl 535 g | | 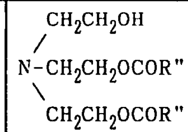 -COR"; resulting from hardened tallow 661 g | | 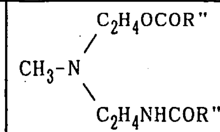 -COR"; resulting from hardened tallow 630 g | | 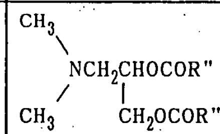 -COR"; resulting from hardened tallow 631 g | |
| Polyhydric alcohol ester | reaction product of Synth. Ex. 4-1 146 g | none | reaction product of Synth. Ex. 4-2 178 g | none | reaction product of Synth. Ex. 4-1 170 g | none | reaction product of Synth. Ex. 4-3 170 g | none |
| Isopropyl alcohol | none | 146 g | none | 178 g | none | 170 g | none | 170 g |
| Quaternizing agent | $CH_3Cl$ 61 g | | $CH_3Cl$ 61 g | | $CH_3Cl$ 61 g | | $CH_3Cl$ 61 g | |
| Reaction temp. | 90°C | | 90°C | | 90°C | | 90°C | |
| Reaction time | 6 hr | | 6 hr | | 6 hr | | 6 hr | |
| Quaternary ammonium salt formed | 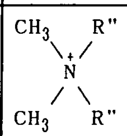 $Cl^-$ | | 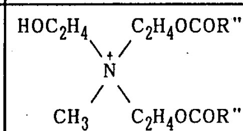 $Cl^-$ | | 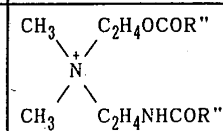 $Cl^-$ | | 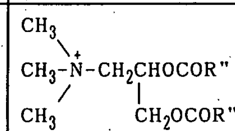 $Cl^-$ | |
| Evaluation of softness | 1 | 0 | 2 | 0 | 4 | 1 | 3 | 1 |
| Evaluation of smell | ○ | × | ○ | × | ○ | × | ○ | × |

It can be understood from the results given in Table 4-2 that the reaction mixtures of Examples are superior to those of Comparative Examples in both softening effect and smell. Further, it can be understood that a remarkably enhanced softening effect is attained in Examples 4-2, 4-3 and 4-4, particularly Example 4-3.

Example 4-5

500 g of the reaction product of Synthesis Example 4-4 and 630 g of diester of tallow fatty acid with N-methyldiethanolamine were charged into a four-necked flask fitted with a stirrer, a thermometer and a condenser. The contents were heated to 70°C to conduct dissolution. 120 g of dimethylsulfuric acid was dropped into the obtained solution in 2 hours and the resulting mixture was aged at that temperature for 3 hours to give an objective ammonium salt.

Examples 4-6 and 4-7

Quaternary ammonium salts listed in Table 4-3 were each prepared in a similar manner to that of Example 4-5 under the conditions specified in Table 4-3.
The reaction conditions of Examples 4-5 to 4-7 and the structures of the obtained quaternary ammonium salts ate given in Table 4-3 together with the results of evaluation of softness and smell.

Table 4-3

| | Ex. 4-5 | Ex. 4-6 | Ex. 4-7 |
|---|---|---|---|
| Tert. amine compound | 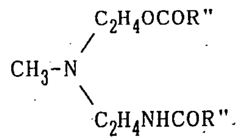 -COR"; resulting from tallow 630 g | 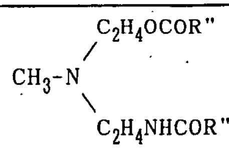 -R"; $C_{17}H_{35}$ 664 g | 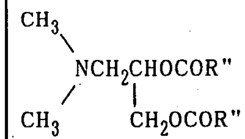 -COR"; resulting from hardened tallow 630 g |
| Polyhydric alcohol ester | reaction product of Synth. Ex. 4-4 500 g | reaction product of Synth. Ex. 4-5 600 g | reaction product of Synth. Ex. 4-6 100 g |
| Alcoholic solvent | none | none | isopropyl alcohol 50 g |
| Quaternizing agent | dimethylsulfuric acid 120 g | dimethylsulfuric acid 120 g | dimethylsulfuric acid 120 g |
| Reaction temp. | 70°C | 60°C | 70°C |
| Reaction time | 5 hr | 8 hr | 6 hr |
| Quaternary ammonium salt formed | 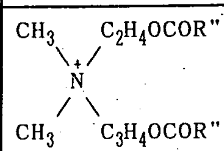 $CH_3SO_4^-$ | 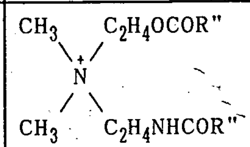 $CH_3SO_4^-$ | 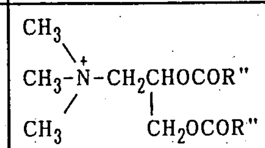 $CH_3SO_4^-$ |
| Evaluation of softness | 2 | 4 | 3 |
| Evaluation of smell | ○ | ○ | ○ |

As apparent from the above results, the softener compositions (1) and (2) of the present invention can impart excellent softness and water absorption properties to cloth without making the cloth greasy to the touch, and are excellent in storage stability.

## Claims

1. A liquid softener composition comprising the following component (2-A) and the following component (2-B) wherein the weight ratio of the component (2-A) to the component (2-B) ranges from 2/1 to 1/9:
   [component (2-A)]
   a component represented by the following general formula (2-I) :

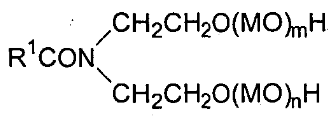

(2-I)

[wherein

$R^1$: a linear or branched alkyl or alkenyl group having 7 to 23 carbon atoms.
M: an alkylene group having 2 or 3 carbon atoms, and m and
n: each a number of 0 or above; provided that the sum of m and n is 0 to 4 on the average].

[component (2-B)]
a compound represented by the following general formula (X):

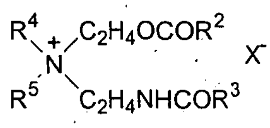

(X)

[wherein

$R^2$ and $R^3$: the same or different from each other a linear or branched alkyl or alkenyl group having 7 to 23 carbon atoms,
$R^4$ and $R^5$: each an alkyl group having 1 to 4 carbon atoms, and
$X^-$ a halogen ion or $R^4SO_4$ wherein $R^4$ is as defined above].

2. A composition as set forth in claim 1, wherein m and n are each 0 in the general formula (2-I).

3. A composition as set forth in claim 1 or 2, wherein $R^1$ is at least one member selected from the group consisting of alkyl group resulting from coconut oil fatty acid, alkyl group resulting from palm kernel oil fatty acid, alkyl group resulting from palm oil fatty acid, alkyl group resulting from palm stearic acid, alkyl group resulting from hardened palm stearic acid, alkyl group resulting from tallow fatty acid and alkyl group resulting from hardened tallow fatty acid in the general formula (2-I).

4. A composition as set forth in claim 1, wherein the total amount of the component (2-A) and the component (2-B) is 3 to 40 % by weight based on the composition.

5. A composition as set forth in claim 1, which further contains the following component (2-C) with the provisos that the amount of the component (2-C) is 0.5 to 5 % by weight based on the composition, that the weight ratio of the component (2-C) to the component (2-B) ranges from 1/100 to 1/2.5 and that the total amount of the component (2-B) and the component (2-C) is 4 to 50 % by weight based on the composition:
   [component (2-C)]

a polyether compound prepared by the addition reaction of a compound having at least three active hydrogen atoms with an alkylene oxide having 2 or 3 carbon atoms (wherein the total amount of oxyethylene group moieties is 55 % by weight or above in the molecule of the component (2-C)) and having an average molecular weight of 5,000 to 2,000,000, or a derivative thereof.

**6.** A composition as set forth in claim 1, which further contains a linear or branched, saturated or unsaturated fatty acid having 8 to 24 carbon atoms as component (2-D) with the provisos that the weight ratio of the component (2-D) to the component (2-B) ranges from 0.5/100 to 1/1 and that the total amount of the component (2-B) and the component (2-D) is 4 to 50 % by weight in the composition.